# EUROPÄISCHE PATENTSCHRIFT

(11) **EP 0 456 069 B1**
(45) Veröffentlichungstag und Bekanntmachung des Hinweises auf die Patenterteilung: **23.10.1996**
(21) Anmeldenummer: 91106893.0
(22) Anmeldetag: 27.04.1991
(51) Int. Cl.: C07D 307/14, C07C 251/34, C07D 333/04, C07D 317/28, C07D 339/06, C07D 309/04, C07D 309/20, C07D 257/02, C07C 239/08, A01N 43/08, A01N 43/10

(54) **Cyclohexenonoximether, Verfahren und Zwischenprodukte zu ihrer Herstellung und ihre Verwendung als Herbizide**
Cyclohexenonoximethers, process and intermediates for their production and their usage as herbicides
Ethers de cyclohexenonoxim, procédé et intermédiaires pour leur production et leur application comme herbicides

(30) Priorität: 09.05.1990 DE 4014983
(43) Veröffentlichungstag der Anmeldung: 13.11.1991
(73) Patentinhaber: BASF Aktiengesellschaft, 67063 Ludwigshafen (DE)
(72) Erfinder: Meyer, Norbert, Dr., W-6802 Ladenburg (DE); Kast, Juergen, Dr., W-6737 Boehl-Iggelheim (DE); Misslitz, Ulf, Dr., W-6730 Neustadt (DE); Harreus, Albrecht, Dr., W-6700 Ludwigshafen (DE); Goetz, Norbert, Dr., W-6520 Worms 1 (DE); Wuerzer, Bruno, Dr., W-6701 Otterstadt (DE); Walter, Helmut, Dr., W-6719 Obrigheim (DE); Westphalen, Karl-Otto, Dr., W-6720 Speyer (DE); Gerber, Matthias, Dr., W-6704 Mutterstadt (DE)

(56) Entgegenhaltungen:
- EP-A- 0 125 094
- EP-A- 0 218 233
- US-A- 4 440 566

## Beschreibung

Die Erfindung betrifft neue herbizid wirksame Cyclohexenonoximether der Formel I 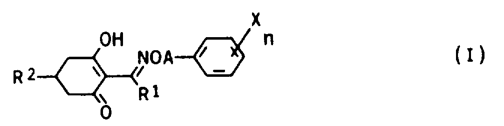 in der die Variablen folgende Bedeutung haben:
- R¹: eine C₁-C₆-Alkylgruppe;
- A: eine C₃-, C₅-, C₆-Alkylen- oder C₃-, C₅-, C₆-Alkenylenkette, wobei diese Ketten ein bis drei C₁-C₃-Alkylgruppen oder eine Methylengruppe tragen können;
- X: Nitro, Cyano, Halogen, C₁-C₄-Alkyl, C₁-C₄-Alkoxy, Phenoxy-C₁-C₄-Alkylthio, C₁-C₄-Halogenalkyl, C₁-C₄-Halogenalkoxy, Carboxyl, C₁-C₄-Alkoxycarbonyl, Benzyloxycarbonyl und/oder Phenyl, wobei die aromatischen Substituenten noch einen bis drei Reste tragen können, ausgewählt aus einer Gruppe bestehend aus Nitro, Cyano, Halogen, C₁-C₄-Alkyl, C₁-C₄-Alkoxy, C₁-C₄-Alkylthio, C₁-C₄-Halogenalkyl, C₁-C₄-Halogenalkyloxy, Carboxyl, C₁-C₄-Alkoxycarbonyl- und Benzyloxycarbonyl, C₁-C₄-Alkoxy-C₁-C₆-alkyl;
- n: 0 bis 3 oder 1 bis 5 für den Fall, daß X Halogen bedeutet;
- R²: eine C₁-C₄-Alkoxy-C₁-C₆-alkyl- oder C₁-C₄-Alkylthio-C₁-C₆-alkylgruppe;
eine C₃-C₇-Cycloalkylgruppe oder eine C₅-C₇-Cycloalkenylgruppe, wobei diese Gruppen einen bis drei Reste tragen können, ausgewählt aus einer Gruppe bestehend aus C₁-C₄-Alkyl, C₁-C₄-Alkoxy, C₁-C₄-Alkylthio, C₁-C₄-Halogenalkyl, Hydroxy und Halogen;
ein 5-gliedriger gesättigter Heterocyclus der ein oder zwei Heteroatome enthält, ausgewählt aus einer Gruppe bestehend aus Sauerstoff und Schwefel, und der noch einen bis drei Reste tragen kann, ausgewählt aus einer Gruppe bestehend aus C₁-C₄-Alkyl, C₁-C₄-Alkoxy, C₁-C₄-Alkylthio und C₁-C₄-Halogenalkyl;
ein gesättigter oder partiell oder vollständig ungesättigter 6- oder 7-gliedriger Heterocyclus, enthaltend ein oder zwei Heteroatome, ausgewählt aus einer Gruppe bestehend aus Sauerstoff und Schwefel, wobei der Heterocyclus noch einen bis drei Reste tragen kann, ausgewählt aus einer Gruppe bestehend aus Hydroxy, Halogen, C₁-C₄-Alkyl, C₁-C₄-Alkoxy, C₁-C₄-Alkylthio und C₁-C₄-Halogenalkyl,
ein 5-gliedriger Heteroaromat, enthaltend ein bis zwei Stickstoffatome und ein Sauerstoffatom oder ein Schwefelatom, wobei dieser Ring noch einen bis drei Reste tragen kann, ausgewählt aus einer Gruppe bestehend aus Halogen, Cyano, C₁-C₄-Alkyl, C₁-C₄-Alkoxy. C₁-C₄-Alkylthio, C₁-C₄-Halogenalkyl, C₂-C₆-Alkenyl, C₂-C₆-Alkenyloxy, C₂-C₆-Halogenalkenyl und C₁-C₄-Alkoxy-C₁-C₄-alkyl,
die Phenyl- oder die Pyridylgruppe, wobei diese Gruppen noch einen bis drei Reste tragen können, ausgewählt aus einer Gruppe bestehend aus Halogen, Nitro, Cyano, C₁-C₄-Alkyl, C₁-C₄-Alkoxy, C₁-C₄-Alkylthio, C₁-C₄-Halogenalkyl, C₃-C₆-Alkenyloxy, C₃-C₆-Alkinyloxy und -NR³R⁴, worin
- R³: Wasserstoff, eine C₁-C₄-Alkylgruppe, eine C₃-C₆-Alkenylgruppe oder eine C₃-C₆-Alkinylgruppe und
- R⁴: Wasserstoff, eine C₁-C₄-Alkylgruppe, eine C₃-C₆-Alkenylgruppe, eine C₃-C₆-Alkinylgruppe, eine C₁-C₆-Acylgruppe oder ein Benzoylrest, wobei der aromatische Ring noch einen bis drei Reste tragen kann, ausgewählt aus einer Gruppe bestehend aus Nitro, Cyano, Halogen, C₁-C₄-Alkyl, C₁-C₄-Alkoxy, C₁-C₄-Alkylthio und C₁-C₄-Halogenalkyl
bedeuten,
sowie ihre landwirtschaftlich nutzbaren Salze und Ester von C₁-C₁₀-Carbonsäuren und anorganischen Säuren.

Außerdem betrifft die Erfindung ein Verfahren und Zwischenprodukte zu ihrer Herstellung sowie ihre Anwendung als Pflanzenschutzmittel.

Die erfindungsgemäßen Cyclohexenone I haben offensichtlich sauren Charakter, d.h. sie können einfache Umsetzungsprodukte wie Salze von Akali- oder Erdalkaliverbindungen oder Enolester bilden.

Die Verbindungen der Formel I können in mehreren tautomeren Formen auftreten, die alle vom Anspruch erfaßt werden.

In der Literatur sind Cyclohexenone der allgemeinen Formel I'

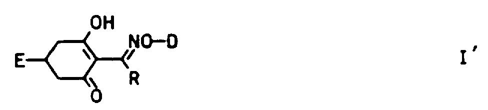

in der u.a.
- D Benzyl und E 2-Ethylthiopropyl (US-A 4 440 566);
- D Benzyl, But-2-enyl und E einen substituierten 5-gliedrigen Heteroarylrest (EP-A 238 021, EP-A 125 094);
- D Benzyl, But-2-enyl und E ein substituiertes Phenyl (EP-A 80 301);
- D But-2-enyl und E einen 5- bis 7-gliedrigen heterocyclischen Ring mit bis zu zwei O-, S-Atomen und mit bis zu zwei Doppelbindungen (EP-A 218 233)
bedeutet, als Herbizide beschrieben.

Es werden jedoch Verbindungen gesucht, die bei geringer Aufwandmenge hohe Selektivität aufweisen, d.h. unerwünschte Pflanzen bekämpfen, ohne dabei die Kulturpflanzen zu schädigen.

Entsprechend dieser Aufgabe wurden die neuen Cyclohexenonoximether der Formel I gefunden, die eine gute herbizide Wirkung, vorzugsweise gegen Arten aus der Familie der Gräser (Gramineen) besitzen. Sie sind verträglich und somit selektiv in breitblättrigen Kulturen sowie in monokotylen Gewächsen, welche nicht zu den Gramineen zählen. Ferner sind Verbindungen darunter, welche sich auch in Gramineenkulturen wie Reis, Mais oder Weizen selektiv verhalten und gleichzeitig unerwünschte Gräser bekämpfen.

Die Cyclohexenone der Formel I können in an sich bekannter Weise aus schon bekannten Derivaten der Formel II (EP-A 80 301, EP-A-125 094, EP-A 142 741, US-A 4 249 937, EP-A 137 174 und EP-A 177 913) und den entsprechenden Hydroxylaminen der Formel III (Houben-Weyl, 10/1 S. 1181ff) hergestellt werden (DE-A-34 33 767, EP-A-48 911).

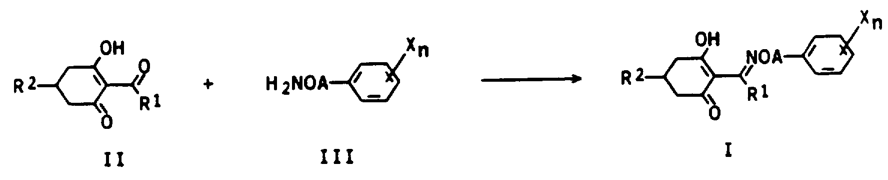

Zweckmäßig führt man die Umsetzung in heterogener Phase in einem Lösungsmittel, bei einer ausreichenden Temperatur unterhalb von etwa 80°C, in Gegenwart einer Base durch und verwendet das Hydroxylamin III in Form seines Ammoniumsalzes.

Geeignete Basen sind z. B. Carbonate, Hydrogencarbonate, Acetate, Alkoholate oder Oxide von Alkali- oder Erdalkalimetallen, insbesondere Natriumhydroxid, Kaliumhydroxid, Magnesiumoxid, Calciumoxid. Außerdem können organische Basen wie Pyridin oder tertiäre Amine Verwendung finden. Die Base wird beispielsweise in einer Menge von 0,5 bis 2 Mol-Äquivalent bezogen auf die Ammoniumverbindung zugesetzt.

Als Lösungsmittel eignen sich beispielsweise Dimethylsulfoxid; Alkohole wie Methanol, Ethanol und Isopropanol; aromatische Kohlenwasserstoffe wie Benzol und Toluol; chlorierte Kohlenwasserstoffe wie Chloroform und Dichlorethan; aliphatische Kohlenwasserstoffe wie Hexan und Cyclohexan; Ester wie Essigsäureethylester und Ether wie Diethylether, Dioxan und Tetrahydrofuran. Vorzugsweise führt man die Umsetzung in Methanol mit Natriumhydrogencarbonat als Base durch.

Die Reaktion ist nach wenigen Stunden beendet. Die Zielverbindung kann z.B. durch Einengen der Mischung, Verteilung des Rückstandes in Methylenchlorid/Wasser und Abdestillieren des Lösungsmittels unter vermindertem Druck isoliert werden.

Man kann für diese Umsetzung aber auch unmittelbar die freie Hydroxylaminbase, z.B. in Form einer wäßrigen Lösung, verwenden; je nach verwendetem Lösungsmittel für die Verbindung II erhält man ein ein- oder zweiphasiges Reaktionsgemisch.

Geeignete Lösungsmittel für diese Variante sind beispielsweise Alkohole wie Methanol, Ethanol, Isopropanol und Cyclohexanol; aliphatische und aromatische, gegebenenfalls chlorierte Kohlenwasserstoffe wie Hexan, Cyclohexan, Methylenchlorid, Toluol und Dichlorethan; Ester wie Essigsäureethylester; Nitrile wie Acetonitril und cyclische Ether wie Dioxan und Tetrahydrofuran.

Alkalimetallsalze der Verbindungen I können durch Behandeln der 3-Hydroxyverbindungen mit Natrium- oder Kaliumhydroxid bzw. -alkoholat in wäßriger Lösung oder in einem organischen Lösungsmittel wie Methanol, Ethanol, Aceton und Toluol erhalten werden.

Andere Metallsalze wie Mangan-, Kupfer, Zink-, Eisen-, Calcium-, Magnesium- und Bariumsalze können aus den Natriumsalzen in üblicher Weise hergestellt werden, ebenso Ammonium- und Phosphoniumsalze mittels Ammoniak, Phosphonium-, Sulfonium- oder Sulfoxoniumhydroxiden.

Die Verbindungen des Typs II können z.B. aus den entsprechenden Cyclohexan-1,3-dionen der Formel IV 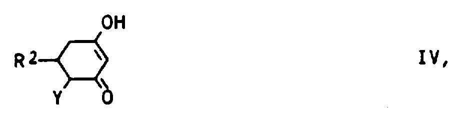 in der
- Y: Wasserstoff oder Methoxycarbonyl bedeutet
nach bekannten Methoden (Tetrahedron Lett., 2491 (1975)) hergestellt werden.

Es ist auch möglich, die Verbindungen der Formel II über die Zwischenstufe der Enolester V herzustellen, die bei der Umsetzung von Verbindungen der Formel IV mit Säurechloriden VII in Gegenwart von Basen anfallen und anschließend mit bestimmten Imidazol- oder Pyridinderivaten umgelagert werden (JP-OS 79/063 052).

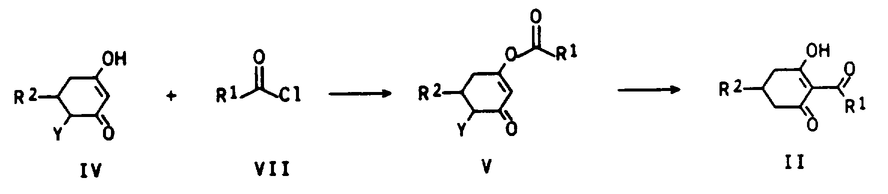

Zu den Verbindungen der Formel IV gelangt man über eine Reihe bekannter Verfahrensschritte ausgehend von bekannten Vorprodukten.

Die Hydroxylamine der Formel III 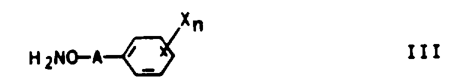 können nach bekannten Verfahren, z.B. wie in Houben-Weyl, Methoden der organischen Chemie, Bd. 10/1, Seite 1181ff beschrieben, hergestellt werden. Besonders bevorzugt ist die in der älteren europäischen Anmeldung Nr. 244 786 beschriebene Verfahrensweise, wenn A für eine Alkenylenkette steht. Danach geht man aus von Verbindungen der Formel VI 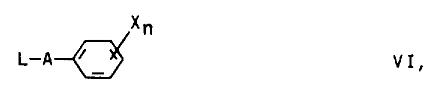 worin L eine unter nukleophilen Bedingungen verdrängbare Abgangsgruppe ist und A eine C₃-, C₅- oder C₆-Alkylengruppe bedeutet, in der die Doppelbindung entweder in Konjugation mit dem aromatischen System steht oder von diesem durch 1 oder 2 Kohlenstoffatome getrennt ist. Als Substituenten der Alkylengruppe können insbesondere Methyl, Chlor oder Fluor in Betracht. Xₙ hat die für die Oximether I genannten Bedeutungen.

Die Verbindungen VI werden zunächst umgesetzt mit einem Hydroxyimid der Formel VIII 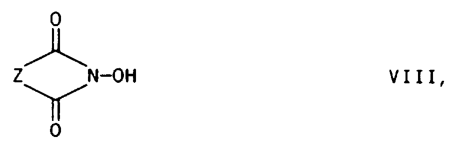 in der Z für einen Phenylen-, Naphthylen, Pyridinylen-, Cyclopentylen-, Cyclohexylen-, Cyclohexenylen-, C₂- bis C₄-Alkenylen- oder C₁- bis C₄-Alkylenrest steht, wobei diese Reste Z unsubstituiert sind oder 1, 2, 3 oder 4 Halogen-, C₁- bis C₄-Alkyl- und/oder C₁- bis C₄-Halogenalkyl-Substituenten tragen können, in Gegenwart eines Lösungsmittels und einer Base bei Temperaturen von 0 bis 140°C umsetzt und aus den erhaltenen Imidethern die Hydroxylamine III mittels Basen oder Säuren freisetzt.

Steht die Gruppe Z für einen cyclischen, aromatischen oder heteroaromatischen Rest, so versteht es sich von selbst, daß es sich bei VIII um die Dicarbonsäureimidgruppe solcher Dicarbonsäuren handelt, in denen die Carboxylgruppen in 1,2-Stellung zueinander stehen. Unter Naphthylen- sind somit die Reste 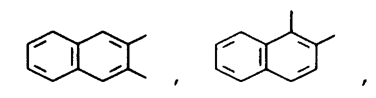 unter Pyridinylen- die Reste 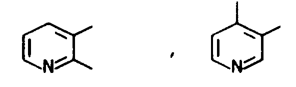 und unter Cyclohexenylen- die Reste 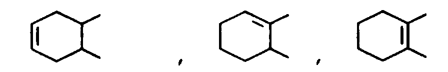 zu verstehen.

Sind die Reste Z substituiert, so kann das Substitutionsmuster beliebig sein. Bevorzugt sind jedoch unsubstituierte Reste Z. Besonders bevorzugt aufgrund der leichten und kostengünstigen Verfügbarkeit der Ausgangsmaterialien sind die Hydroxylaminderivate, in denen Z eine C₂- bis C₃-Alkylen, C₂- bis C₄-Alkenylen- und insbesondere eine Phenylengruppe ist.

Sowohl die Hydroxylaminderivate in denen die Aminogruppe durch eine Dicarbonsäureimidgruppe geschützt ist als auch die eine freie Aminogruppe enthaltenden Hydroxylaminderivate sind stabile Verbindungen und können als solche isoliert, gelagert und weiterverarbeitet werden. Zur Isolierung der Hydroxylaminderivate mit freier Aminogruppe kann es sich jedoch als vorteilhaft erweisen, wenn diese in ihre Salze mit organischen oder anorganischen Säuren überführt werden, da diese Salze leichter kristallin zu erhalten sind. Darüberhinaus kann durch die Wahl des Säureanions das Löslichkeitsverhalten dieser Hydroxylammoniumsalze in organischen Lösungsmitteln oder Wasser gezielt beeinflußt werden - eine Maßnahme, welche die Weiterverarbeitung der erfindungsgemäßen Hydroxylaminderivate erleichtert. Besonders bevorzugt werden die aus den entsprechenden Hydroxylaminderivaten hergestellten Hydroxylammoniumsalze mit Anionen wie Chlorid, Bromid, Sulfat, Nitrat, Phosphat, Formiat, Acetat, Malonat, Oxalat, Methansulfonat, Benzolsulfonat und Toluolsulfonat genutzt.

Die Ausgangsverbindungen VI sind literaturbekanntt oder lassen sich entsprechend dort beschriebenen Verfahren herstellen (z.B. Org. Synth. Coll. Vol. V, 249 cit. lit; EP 48911, EP 143952, US 4 686 735). Die für die Umsetzung bevorzugten Bromide (VI, L = Br) lassen sich aus den entsprechenden Alkoholen nach allgemeinen Vorschriften (z.B. Houben-Weyl, Bd. 5/4, S. 354 ff) synthetisieren. Zur Herstellung der bevorzugten Verbindungen VI, in denen A eine Prop-2-enylengruppe bedeutet, reduziert man Zimtsäureester oder Zimtaldehyde (z.B. Organikum, S. 508 ff) nach allgemein üblichen Verfahren und überführt die so erhalenen Zimtalkohole wie oben beschrieben in die entsprechenden Bromide.

Die Herstellung der Hydroxylamine kann durch folgendes Reaktionsschema dargestellt werden:

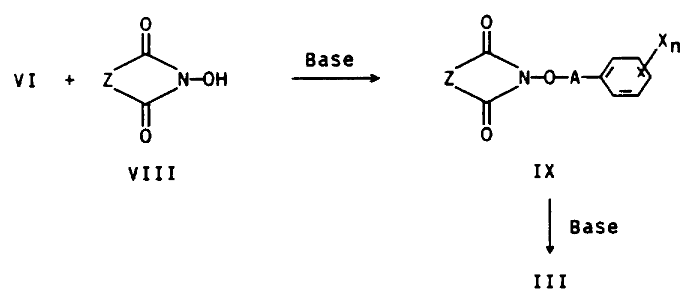

Bevorzugte Abgangsgruppen L sind die Halogenide Chlorid, Bromid und Jodid und die Sulfonsäureester der Methansulfonsäure, Trifluormethansulfonsäure, Benzolsulfonsäure, Brombenzolsulfonsäure oder der Toluolsulfonsäure. Besonders bevorzugte Abgangsgruppen sind die Halogenide Chlorid und Bromid sowie Methansulfonat und Toluolsulfonat.

Die Umsetzung der Ausgangsverbindungen VI mit den Hydroxyimiden wird zweckmäßigerweise in Gegenwart einer Base ausgeführt. Geeignet sind prinzipiell alle Basen, die in der Lage sind, die Hydroxyimide VIII zu deprotonieren ohne das Imidsystem anzugreifen. Dies sind insbesondere die sogenannten nicht-nucleophilen Basen. Beispielsweise genannt seien Mineralbasen wie Alkalimetall- und Erdalkalimetallcarbonate, Alkalimetall-und Erdalkalimetallhydrogencarbnate, organische Basen wie aliphatische, cycloaliphatische und aromatische tertiäre Amine. Es können auch Gemische dieser Basen verwendet werden.

Als Einzelverbindungen seien folgende Basen beispielhaft aufgeführt: Natriumcarbonat, Kaliumcarbonat, Magnesiumcarbonat, Calciumcarbonat, Bariumcarbonat, die Hydrogencarbonate dieser Metalle, Trimethylamin, Triethylamin, Tributylamin, Ethyldiisopropylamin, N,N-Dimethylanilin, 4-N,N-Dimethylaminopyridin, Diazobicyclooctan, Diazobicycloundecan, N-Methylpiperidin, 1,4-Dimethylpiperazin, Pyridin, Chinolin, Bipyridin, Phenanthrolin. Bevorzugt sind die preiswerten Basen Natrium- und Kaliumcarbonat.

Die Base wird im allgemeinen in äquivalenten Mengen bis zu einem Überschuß von 5 Äquivalenten, bezogen auf das Hydroxyimid, zugegeben. Ein höherer Überschuß ist möglich, entbehrt aber zusätzliche Vorteile. Die Verwendung einer geringen Basenmenge ist ebenfalls möglich. Bevorzugt wird jedoch eine Basenmenge von 1 bis 3, insbesondere von 1 bis 2 Äquivalenten, bezogen auf das Hydroxyimid VIII eingesetzt.

Die Verwendung von nucleophilen Basen, z.B. Alkalimetall- und Erdalkalimetallhydroxiden, insbesondere Natrium- und Kaliumhydroxid, ist ebenfalls möglich. In diesem Falle ist es vorteilhaft, die Base in äquivalenten Mengen bezüglich des Hydroxyimids VI einzusetzen, um einem nucleophilen Angriff der Hydroxylionen auf die Carbonylfunktion der Imidgruppierung vorzubeugen.

Zweckmäßigerweise setzt man die Ausgangsverbindungen VI mit den Hydroxyimiden VIII in einem Lösungsmittel um, das sich unter den Reaktionsbedingungen inert verhält. Vorteilhafte Lösungsmittel sind z.B. polareaprotische Lösungsmittel wie Dimethylformamid, N-Methylpyrrolidon, Dimethylsulfoxid, Sulfolan und cyclische Harnstoffe. Die Lösungsmittelmenge ist im allgemeinen nicht kritisch.

Die Umsetzung der Ausgangsverbindungen VI mit den Hydroxyimiden VIII kann auch unter Anwendung der Phasentransfer-Katalyse ausgeführt werden. In diesem Falle werden mit Wasser zwei Phasen bildende Lösungsmittel, bevorzugt Chlorkohlenwasserstoffe, eingesetzt. Als Phasentransferkatalysatoren eignen sich die üblicherweise zu solchen Zwecken verwendeten quartären Ammonium- und Phosphoniumsalze, Polyethylenglykole, Polyethylenglykolether und Kronenether, wie sie z.B. in Dehmlow et al., Phase Transfer Catalysis, S. 37 - 45 und S. 86 - 93, Verlag Chemie, Weinheim 1980, beschrieben sind. Die Phasentransferkatalysatoren werden zweckmäßigerweise in Mengen von 1 bis 10 Vol%, bevorzugt in Mengen von 3 bis 5 Vol%, bezogen auf das Volumen der Reaktionsmischung, eingesetzt.

Die Umsetzung der Ausgangsverbindungen VI mit den Hydroxyimiden VIII wird im allgemeinen im Temperaturbereich zwischen 0 und 140°C, bevorzugt zwischen 20 und 100°C, insbesondere zwischen 40 und 80°C, durchgeführt. Zweckmäßigerweise wird dabei so vorgegangen, daß man das Hydroxyimid VIII zusammen mit der Base im Lösungsmittel vorlegt und das Ausgangsmaterial VI zu dieser Lösung dosiert. Dabei kann es sich als günstig erweisen, wenn das Hydroxyimid bei einer tieferen Temperatur, bespielsweise bei 0 bis 50°C, zugegeben und die Reaktionsmischung erst nach dieser Zugabe auf die eigentliche Reaktionstemperatur erhitzt wird.

Nach beendeter Reaktion wird die abgekühlte Reaktionsmischung zweckmäßigerweise mit Wasser versetzt, wobei sich die gebildeten Hydroxylaminderivate IX als kristalline Festkörper oder als Öle abscheiden. Die auf diese Weise erhaltenen Hydroxylaminderivate können, falls gewünscht, durch Umkristallisation oder durch Extraktion weiter gereinigt werden.

Die Hydroxylaminderivate IX können zwischengelagert werden oder sogleich in die Hydroxylaminderivate mit freier Aminogruppe umgewandelt werden. Diese Umwandlung kann nach an sich bekannten Verfahren durchgeführt werden, wie sie beispielsweise in DE-A 36 15 973 und den darin zitierten Schriften beschrieben sind. Bevorzugt wird das Verfahren gemäß DE-A 36 15 973 angewandt, nach dem die Hydroxylaminderivate III mittels Ethanolamin freigesetzt werden. Die Freisetzung der Hydroxylaminderivate III mit Hilfe anderer Basen wie wäßrigen Mineralbasen, mit Aminen, Hydrazinen, Hydroxylaminen oder mittels wäßriger Säuren ist ebenfalls möglich.

Aus den nach diesen Verfahren erhaltenen Reaktionsgemischen können die Hydroxylaminderivate III mittels üblicher Aufarbeitungsmethoden isoliert werden, beispielsweise durch Extraktion oder durch Kristallisation. Zur Erhöhung der Kristallisationstendenz dieser Hydroxylaminderivate kann es oftmals förderlich sein, diese in ihre Salze mit Mineralsäuren oder organischen Säuren überzuführen. Dazu werden im allgemeinen verdünnte Lösungen dieser Säuren mit den Hydroxylaminderivaten umgesetzt, und zwar zweckmäßigerweise in äquivalenten Mengen. Die erhaltenen Hydroxylammoniumsalze können wie die Hydroxylaminderivate mit freier Aminogruppe direkt zu den Herbiziden der Formel I weiterverarbeitet werden oder auch, falls gewünscht, gelagert werden.

Bevorzugt werden Cyclohexenone der Formel I, in denen die Substituenten folgende Bedeutung haben:
- R¹: eine Alkylgruppe wie Methyl, iso-Propyl, n-Butyl, i-Butyl und t-Butyl, insbesondere aber die Ethyl- und die n-Propylgruppe;
- A: Propylen, Prop-2-enylen, Pentylen, Pent-2-enylen, Pent-3-enylen, Pent-4-enylen, Hexylen, Hex-2-enylen, Hex-3-enylen, Hex-4-enylen, Hex-5-enylen, jeweils gegebenenfalls substituiert durch bis zu 3 Methylreste oder einen Methylenrest; bei den ungesättigten Ketten können sowohl die cis- als auch die trans-Form auftreten. Besonders bevorzugt ist Prop-2-enylen.
- X: Halogen wie Fluor, Chlor, Brom und Iod, insbesondere Fluor und Chlor;
Alkyl wie Methyl, Ethyl, Propyl, 1-Methylethyl, Butyl, 1-Methylpropyl, 2-Methylpropyl und 1,1-Dimethylethyl, insbesondere Methyl,
Alkoxy wie Methoxy, Ethoxy, Propoxy, 1-Methylethoxy, Butoxy, 1-Methylpropoxy, 2-Methylpropoxy und 1,1-Dimethylethoxy, insbesondere Methoxy, Ethoxy, 1-Methylethoxy und 1,1-Dimethylethoxy,
Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio und Ethylthio,
Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Difluormethyl, Trifuormethyl, 2,2,2-Trifluorethyl und Pentafluorethyl,
Halogenalkoxy wie Difluormethoxy, Trifluormethoxy, Chlor-difluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy,
Alkoxycarbonyl wie Methoxycarbonyl, Ethoxycarbonyl, Propyloxycarbonyl, 1-Methylethoxycarbonyl, Butyloxycarbonyl und 1,1-Dimethylethoxycarbonyl, insbesondere Methoxycarbonyl, Ethoxycarbonyl und 1,1-Dimethylethoxycarbonyl, insbesondere Methoxycarbonyl
sowie Wasserstoff, Nitro, Cyano, Benzoxycarbonyl, Phenoxy und Phenyl, wobei die aromatischen Reste substituiert sein können.
Dabei kommen als Substituenten hier besonders Nitro, Cyano, Carboxyl, Benzyloxycarbonyl sowie die obengenannten Halogenatome, Alkylgruppen, Alkoxygruppen, Alkylthiogruppen, Halogenalkylgruppen, Halogenalkoxygruppen und/oder Alkoxycarbonylgruppen in Betracht.

Besonders bevorzugt sind bei diesen aromatischen Resten unsubstituierte oder einfach substituierte Vertreter.
- n: 0, 1, 2 oder 3, insbesondere 0, 1 oder 2. Bei mehreren Resten X können die Substituenten gleich oder verschieden sein.
- R²: Alkyl wie unter R¹ genannt, welches eine der unter X genannten Alkoxy- oder Alkylthiogruppe bevorzugt in 1-, 2- oder 3-Position tragen kann, insbesondere 2-Ethylthiopropyl;
5-gliedriges Heterocycloalkyl wie Tetrahydrofuranyl, Tetrahydrothienyl, Dioxolanyl, Dithiolanyl und Oxathiolanyl, insbesondere Tetrahydrofuranyl und Dioxolanyl, wobei diese Ringe ein bis drei der bereits unter X genannten C₁-C₄-Alkylgruppen, C₁-C₄-Alkoxygruppen, C₁-C₄-Alkylthiogruppen und/oder C₁-C₄-Halogenalkylgruppen tragen können,
5-gliedriges Heteroaryl wie Pyrrolyl, Pyrazolyl, Imidazolyl, Isoxazolyl, Oxazolyl, Isothiazolyl, Thiazolyl, Furanyl und Thienyl, insbesondere Isoxazolyl und Furanyl,
ein 6- oder 7-gliedriger Heterocyclus wie Tetrahydropyran-3-yl, Dihydropyran-3-yl, Tetrahydropyran-4-yl, Dihydropyran-4-yl, Tetrahydrothiopyran-3-yl, Dihydrothiopyran-3-yl, Tetrahydrothiopyran-4-yl, Dihydrothiopyran-4-yl und Dioxepan-5-yl, insbesondere Tetrahydropyran-3-yl, Tetrahydropyran-4-yl und Tetrahydrothiopyran-3-yl,
ein Phenyl- oder ein Pyridylrest,
wobei die cyclischen Reste ein bis drei der unter X genannten Alkylgruppen, Alkoxygruppen, Alkylthiogruppen und/oder Halogenalkylgruppen tragen können.

Die 5-gliedrigen Heteroaromaten in der Bedeutung R² können als Substituenten folgende Reste tragen:
Halogenatom wie unter X genannt, insbesondere Fluor und Chlor,
Alkoxyalkyl wie Methoxymethyl, 2-Methoxyethyl, 2-Methoxypropyl, 3-Methoxypropyl, 2-Methoxy-1-methylethyl, Ethoxymethyl, 2-Ethoxyethyl, 2-Ethoxypropyl, 3-Ethoxypropyl, 2-Ethoxy-1-methylethyl und 1-Ethoxy-1-methylethyl, insbesondere Methoxyethyl und Ethoxyethyl,
Alkenyl wie Ethenyl, 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butnyl, 2-Methyl-3-butenyl, 3-Methyl-3-butenyl, 1,1-Dimethyl-2-propenyl, 1,-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Mthyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, insbesondere 1-Methylethenyl bzw. entsprechendes Alkenyloxy und/oder Halogenalkenyl.

Die 6- und 7-gliedrigen Heterocyclen können neben den vorstehend genannten Substituenten auch Hydroxygruppen tragen.

Bei den Phenyl- und Pyridylresten kommen als Substituenten neben den obengenannten Gruppen auch Reste wie Cyano-, Nitro- und Aminogruppen in Betracht, wobei die Aminogruppen unsubstituiert oder ein- bis zweifach durch die erwähnten Alkyl- und/oder Alkenylgruppen substituiert sein können.

Daneben kommen als Substituenten an Aminogruppen auch folgende Reste in Betracht:
Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-3-butinyl, 2-Methyl-3-butinyl, 1-Methyl-2-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl, insbesondere 2-Propinyl und 2-Butinyl und/oder
Acyl wie Acetyl, Propionyl, Butyryl, 2-Methyl-propionyl, Pentanoyl, 2-Methylbutyryl, 3-Methylbutyryl, 2,2-Dimethylpropionyl, Hexanoyl, 2-Methylpentanoyl, 3-Methylpentanoyl, 4-Methylpentanoyl, 2,2-Dimethylbutyryl, 2,3-Dimethylbutyryl, 3,3-Dimethylbutyryl und 2-Ethylbutyryl, insbesondere Acetyl und Propionyl
oder ein Benzoylrest.

Als Salze der Verbindungen der Formel I kommen landwirtschaftlich brauchbare Salze, beispielsweise Alkalimetallsalze, wie das Kalium- oder Natriumsalz, Erdalkalimetallsalze, wie das Calcium-, Magnesium- oder Bariumsalz, Mangan-, Kupfer-, Zink- oder Eisensalze sowie Ammonium, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Im Hinblick auf die biologische Wirksamkeit seien spezielle Beispiele für diese Cyclohexenonoximether in den folgenden Tabellen zusammengefaßt.

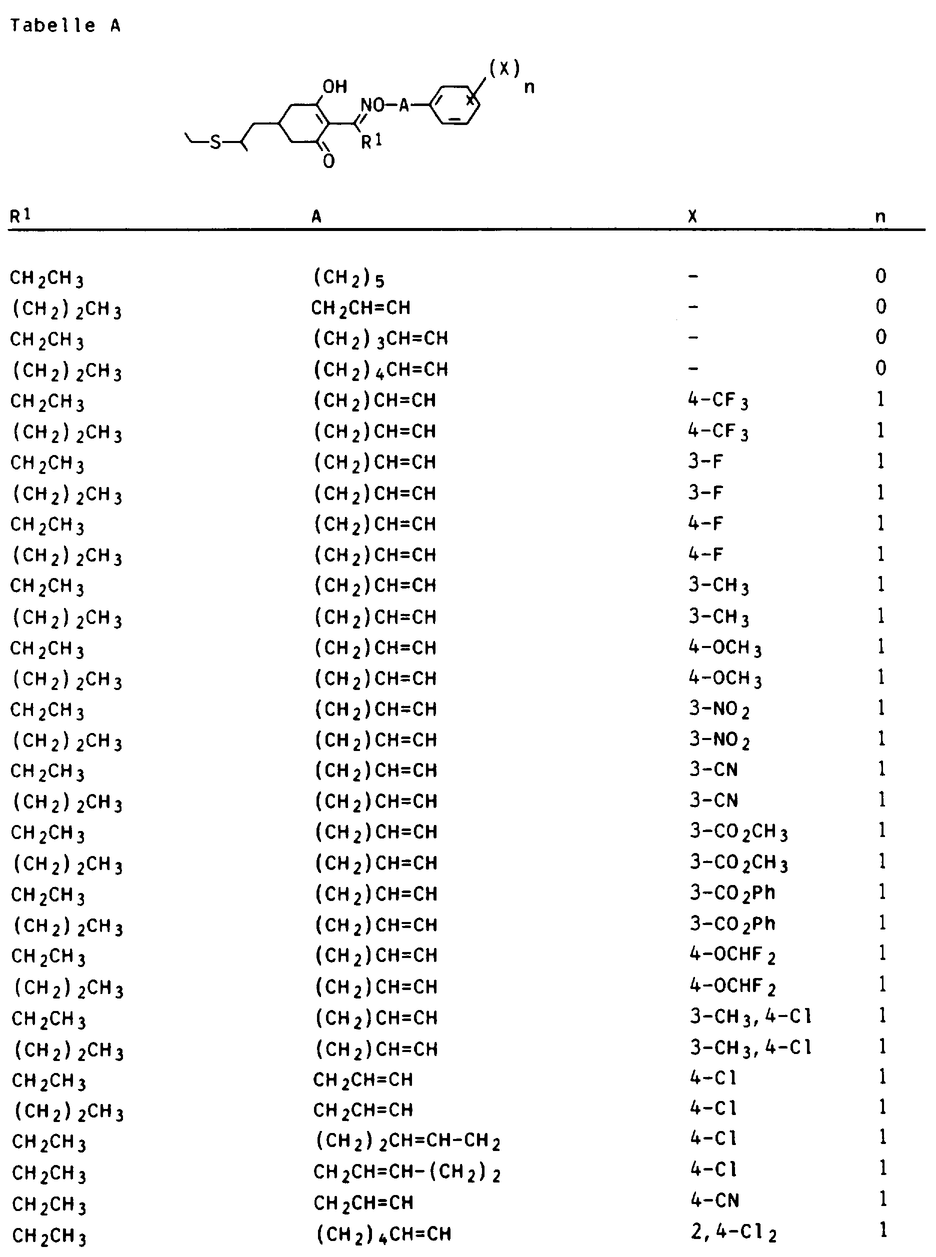

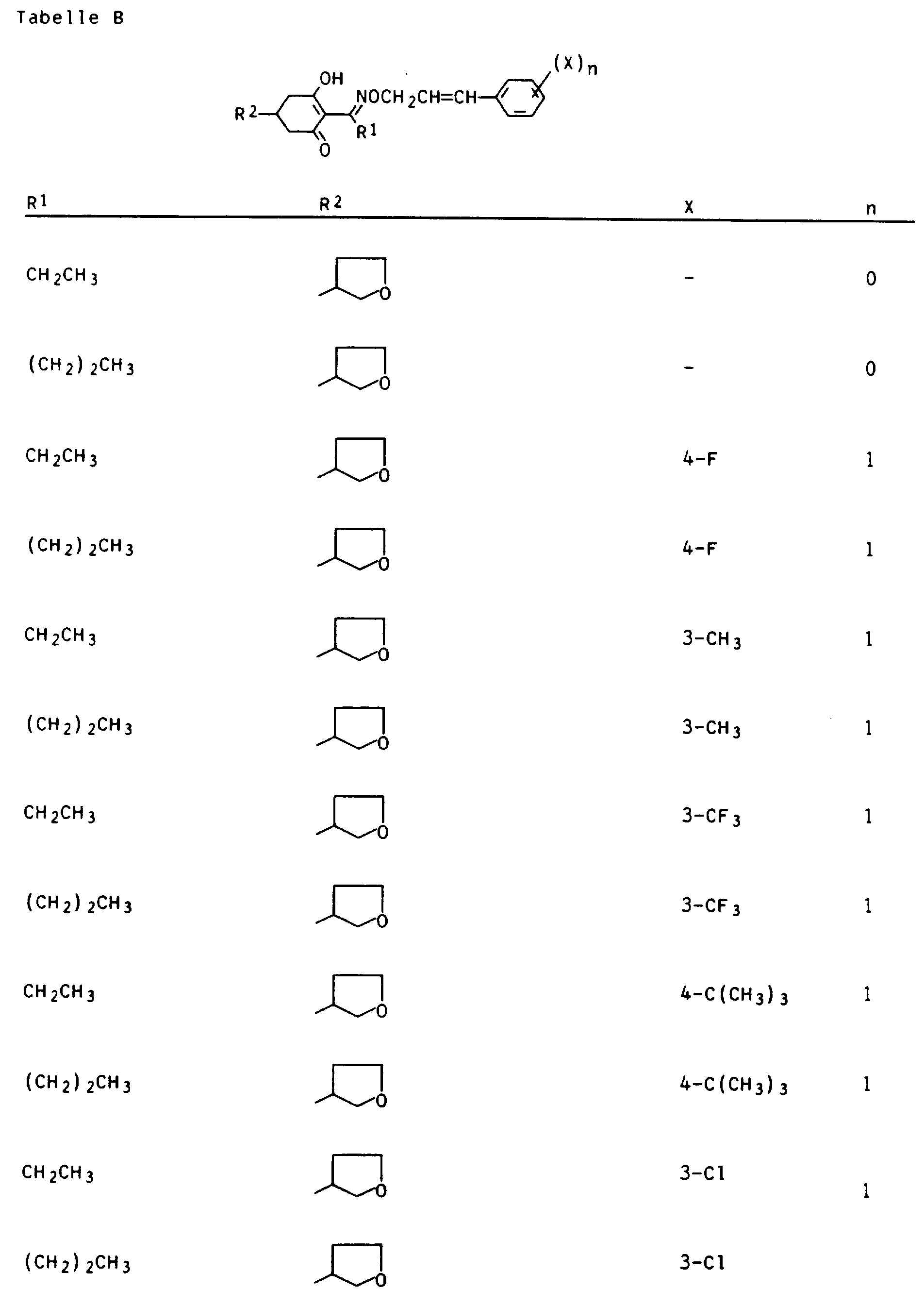

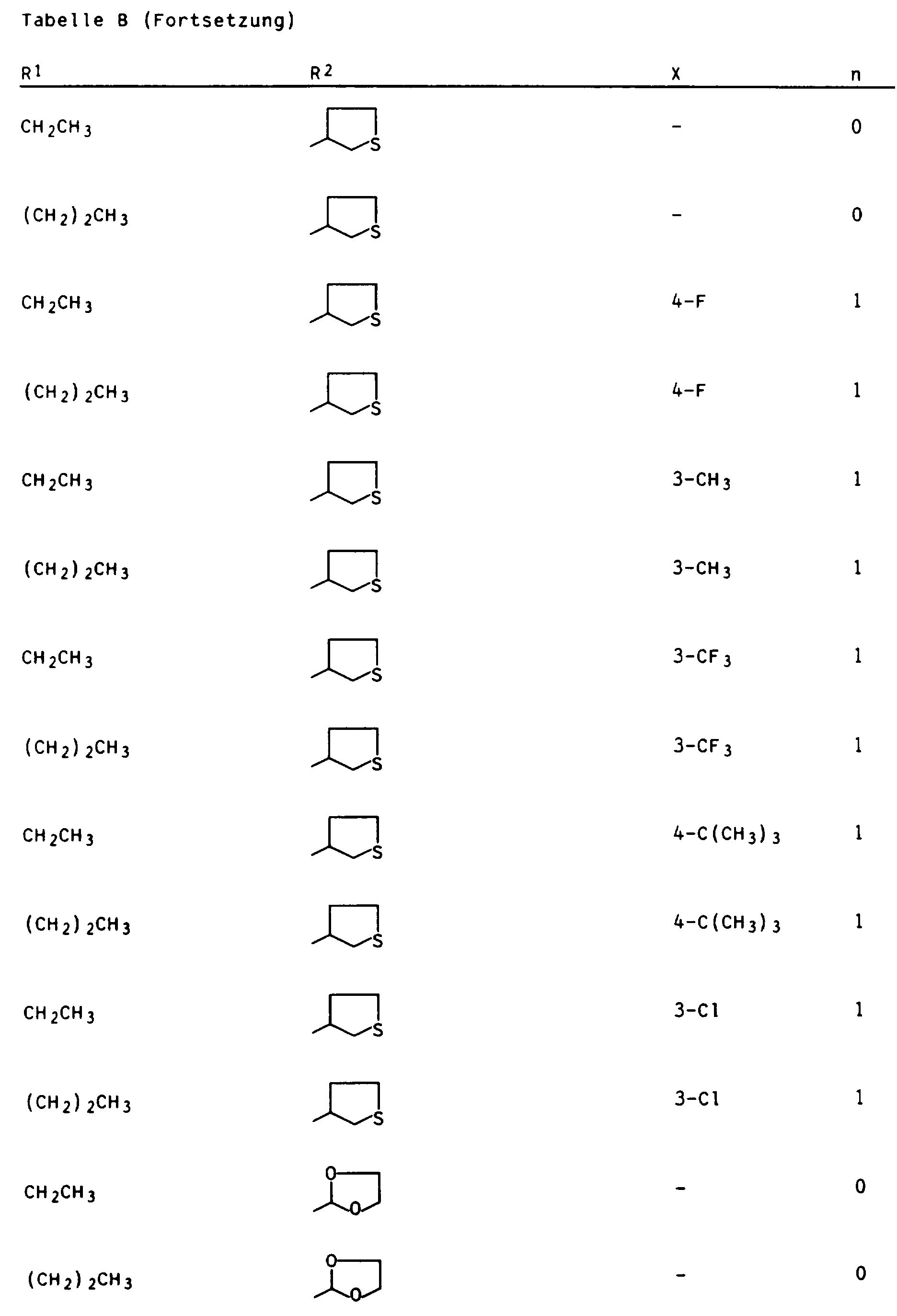

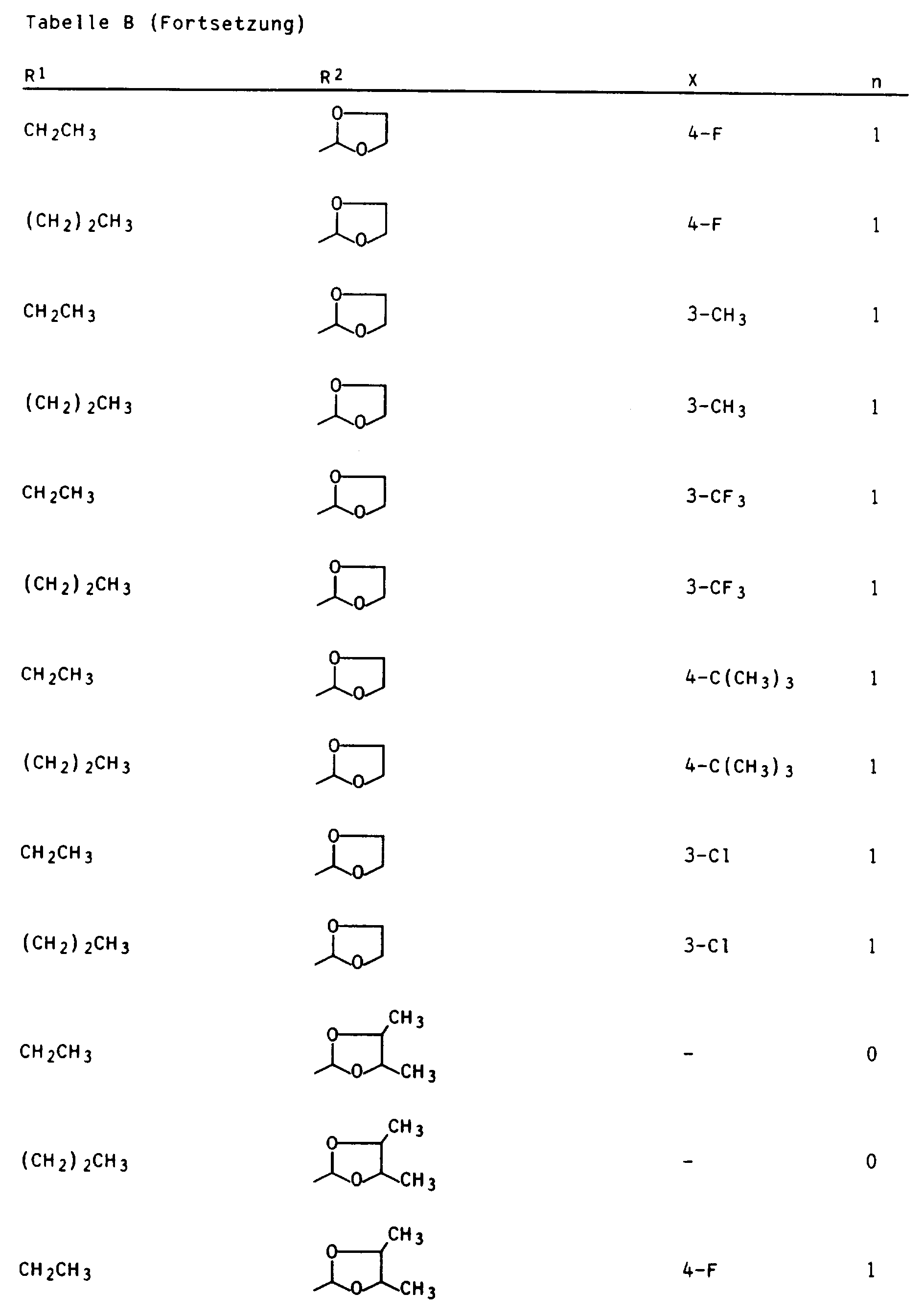

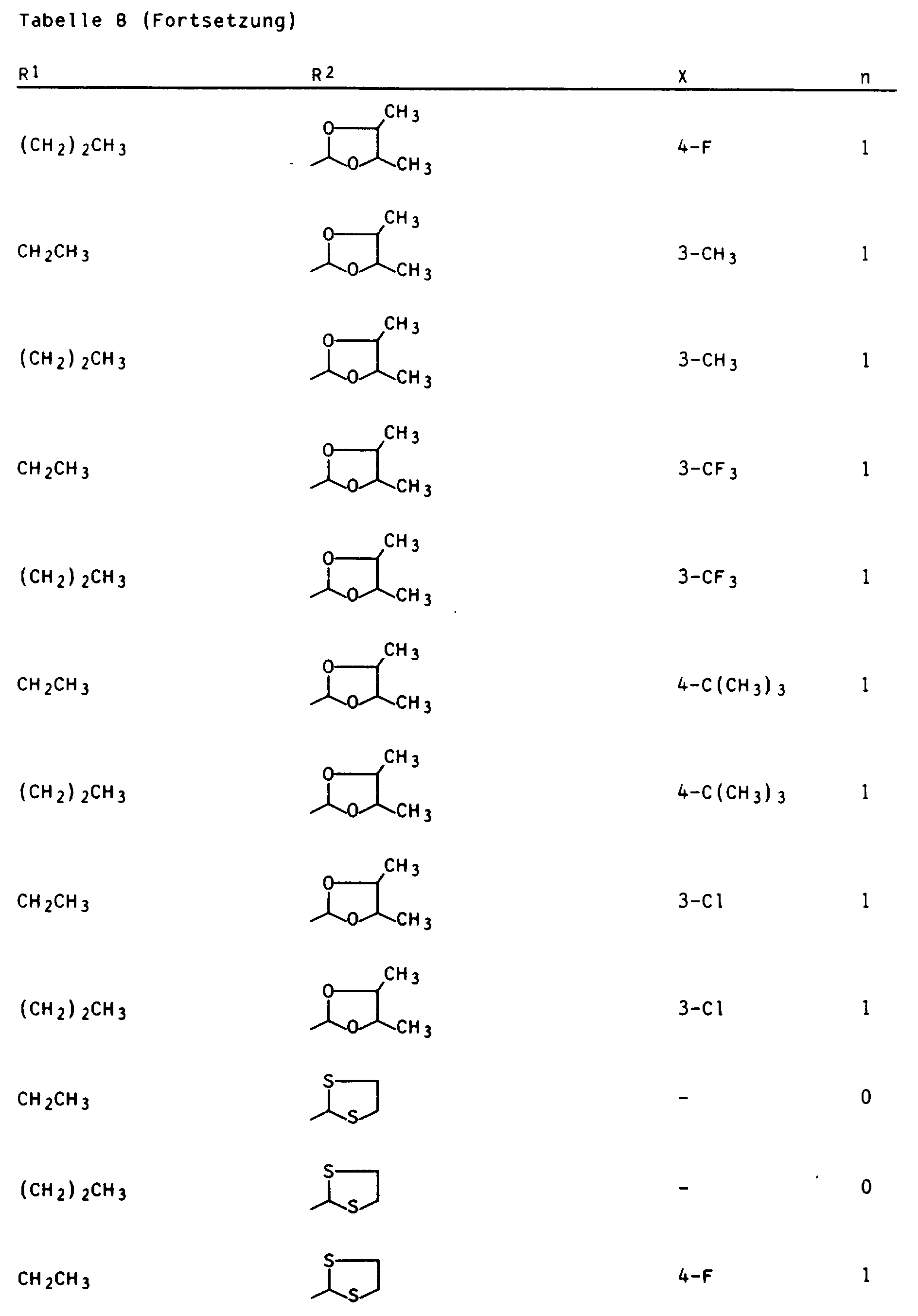

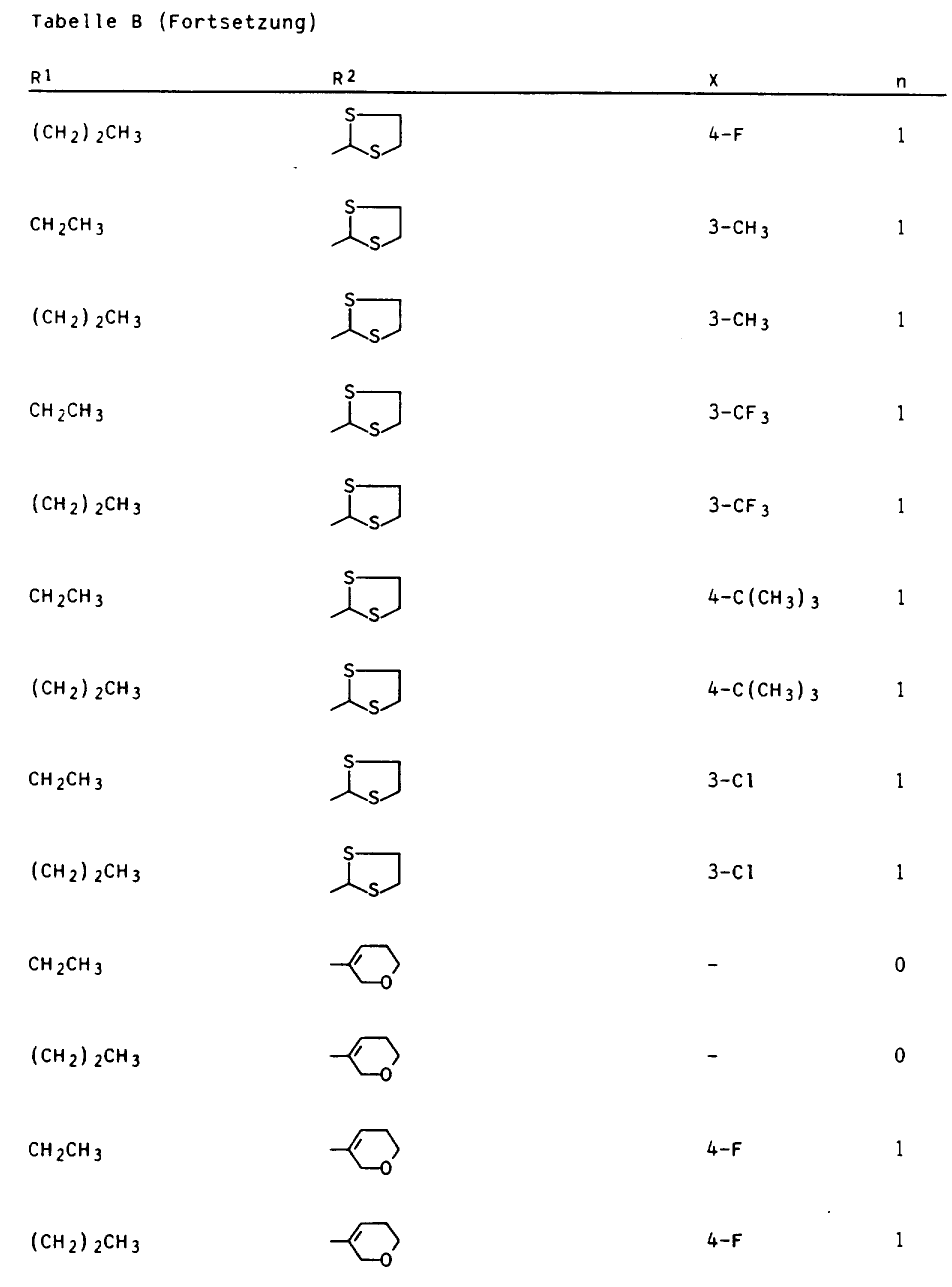

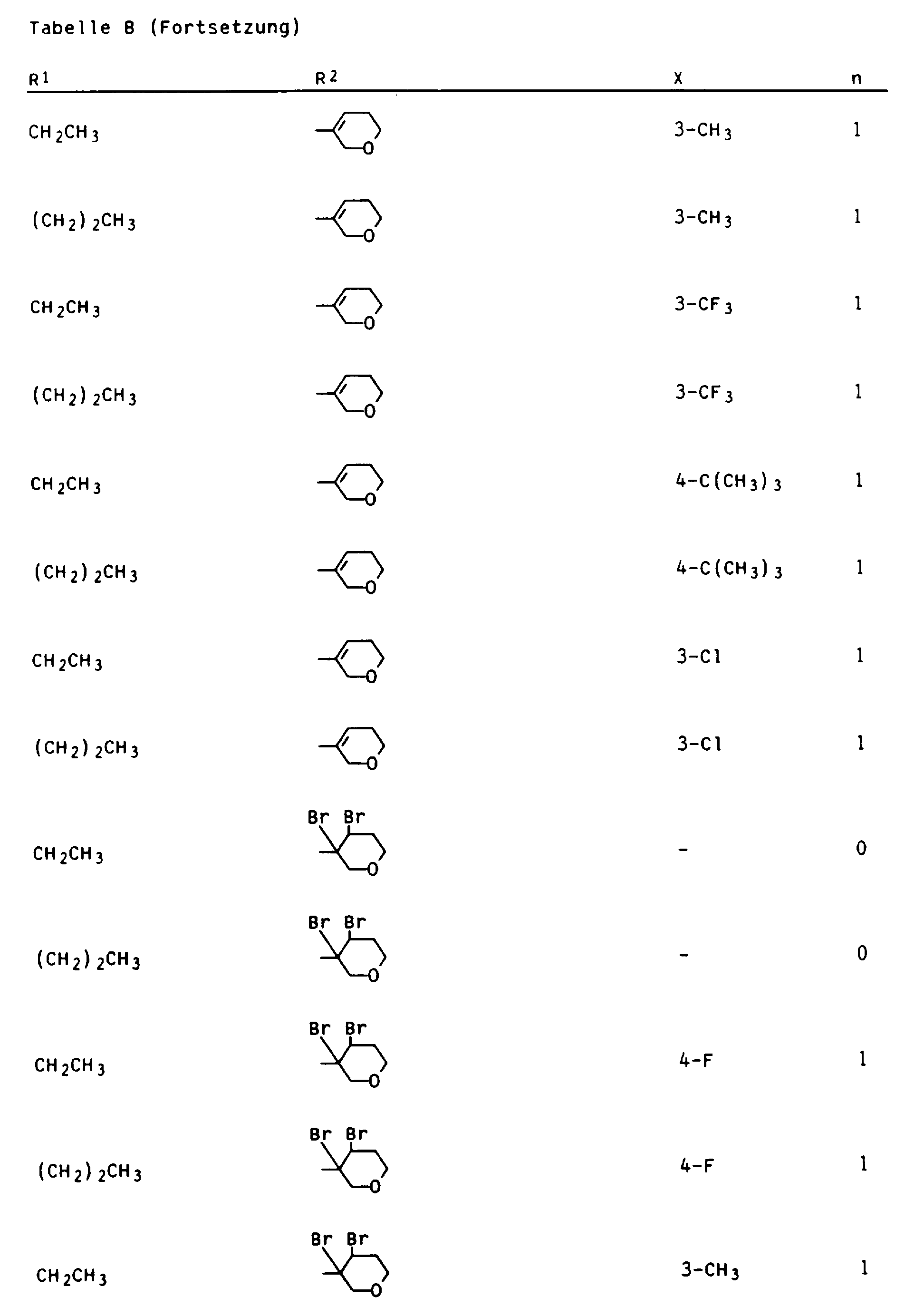

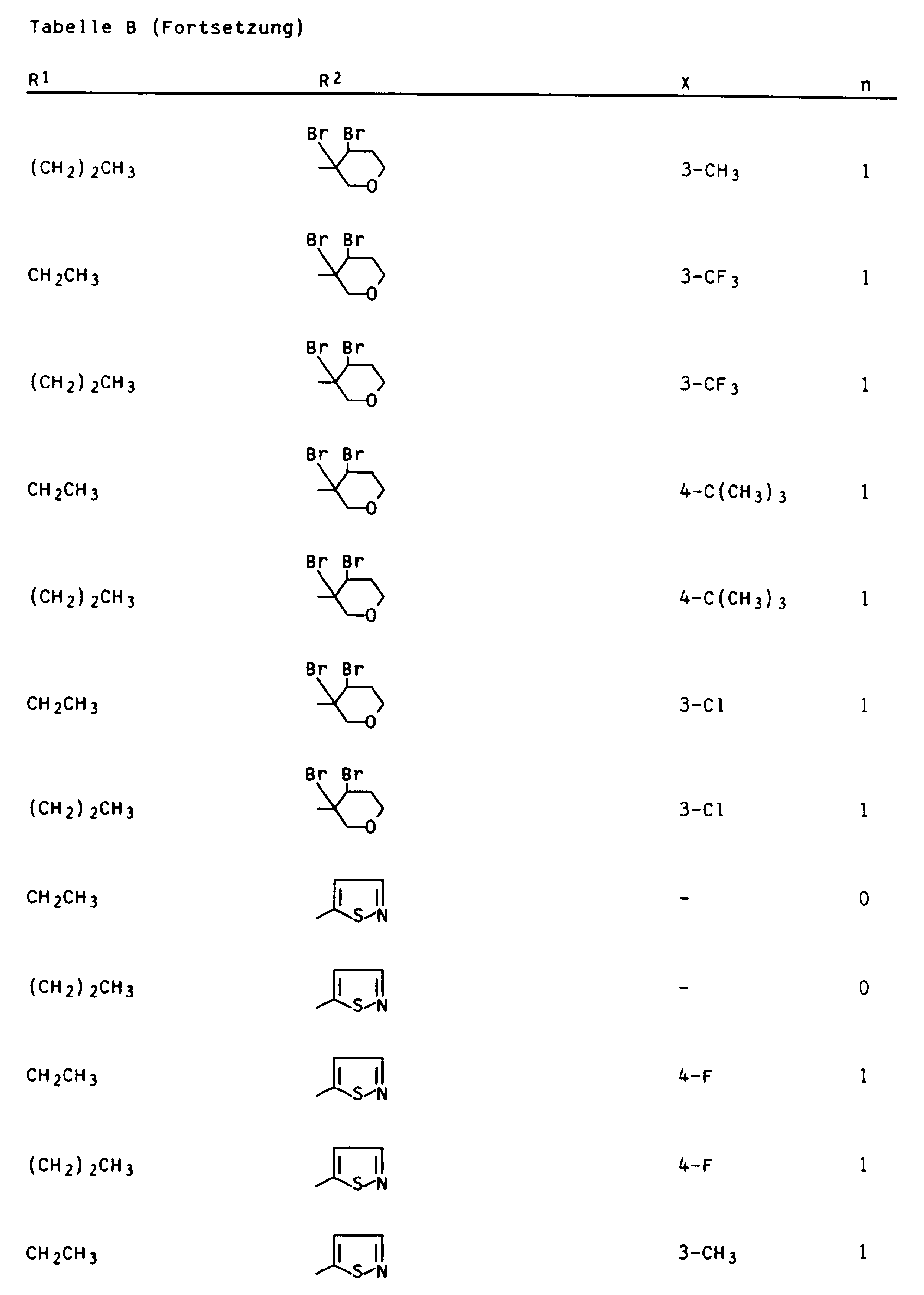

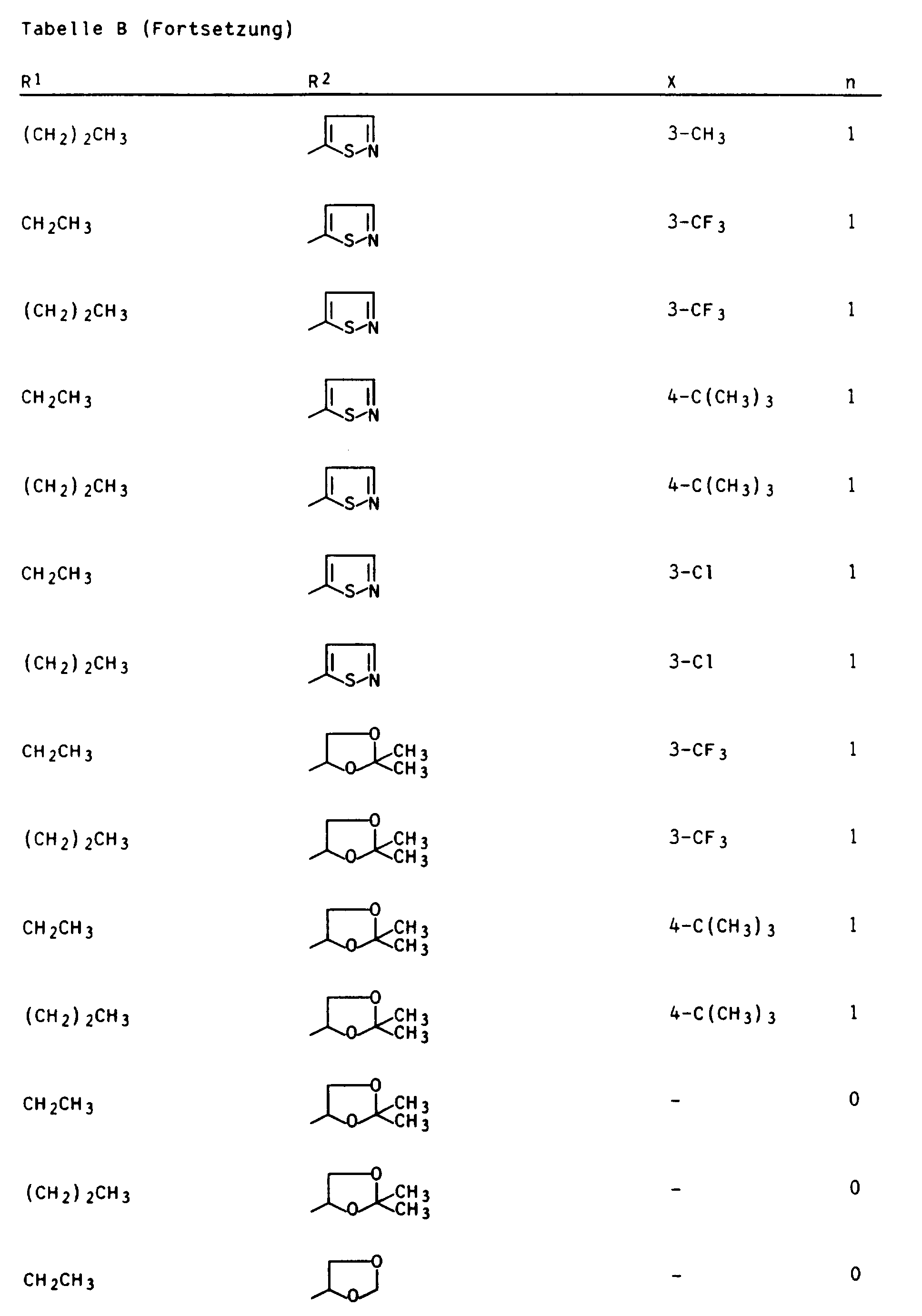

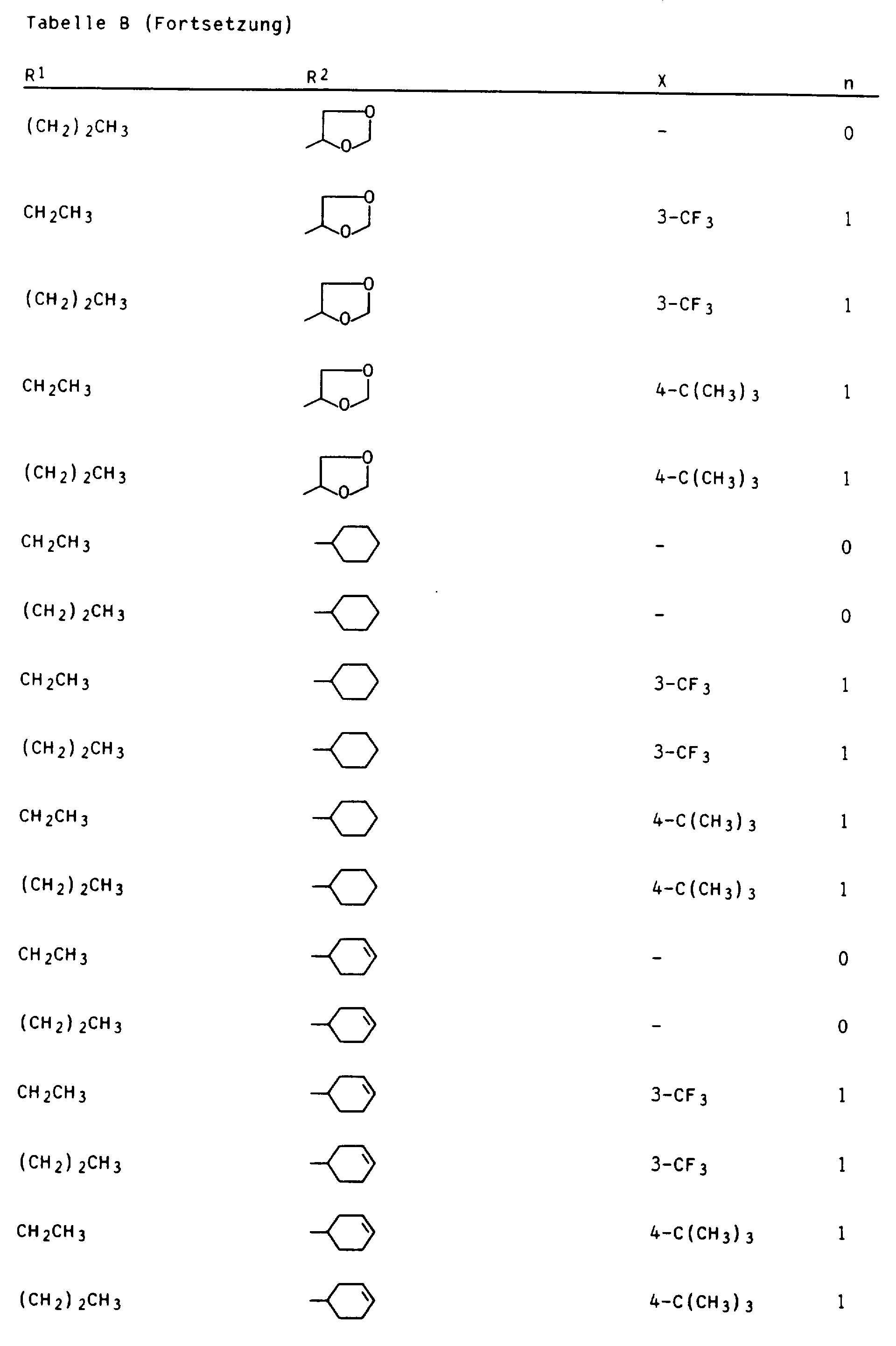

Die Cyclohexenonoximether I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden. Granulate, z.B. Umhüllungs-, Imprägnierungs- und Homogengranulate können durch Bindung der Wirkstoffe an feste Trägerstoffe hergestellt werden Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR/HPLC/GC-Spektrum) eingesetzt.

Beispiele für solche Zubereitungen sind:
I. eine Lösung aus 90 Gew.-Teilen der Verbindung 1.1 und 10 Gew.- Teilen N-Methyl-α-pyrrolidon, die zur Anwendung in Form kleinster Tropfen geeignet ist;
II. eine Mischung aus 20 Gew.-Teilen der Verbindung 1.2, 80 Gew.- Teilen Xylol, 10 Gew.-Teilen des Anlagerungsproduktes von 8 bis 10 Mol Ethylenoxid an 1 Mol Ölsäure-N-monoethanolamid, 5 Gew.- Teilen Calciumsalz der Dodecylbenzolsulfonsäure, 5 Gew.-Teilen des Anlagerungsproduktes und 40 Mol Ethylenoxid an 1 Mol Ricinusöl. Durch feines Verteilen des Gemisches in 100 000 Gew.-Teilen Wasser erhält man eine Dispersion, die 0,02 Gew.-% des Wirkstoffs enthält.
III. eine wäßrige Dispersion aus 20 Gew.-Teilen der Verbindung 1.4, 40 Gew.-Teilen Cyclohexanon, 30 Gew.-Teilen Isobutanol, 20 Gew.- Teilen des Anlagerungsproduktes von 40 mol Ethylenoxid an 1 mol Ricinusöl. Die Mischung dieser Dispersion mit 100 000 Gewichtsteilen Wasser enthält 0,02 Gew.-% des Wirkstoffes.
IV. eine wäßrige Dispersion aus 20 Gew.-Teilen der Verbindung 1.17, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl. Die Mischung dieser Dispersion mit 100 000 Gew.-Teilen Wasser enthält 0,02 % des Wirkstoffes;
V. eine in einer Hammermühle vermahlene Mischung aus 80 Gew.-Teilen der Verbindung 2.2, 3 Gew.-Teilen des Natriumsalzes der Diisobutylnaphtalin-α-sulfonsäure, 10 Gew.-Teilen des Natriumsalzes einer Ligninsulfonsäure aus einer Sulfitablauge und 7 Gew.-Teilen pulverförmigem Kieselsäuregel. Durch feines Verteilen der Mischung in 20 000 Gew.-Teilen Wasser erhält man eine Spritzbrühe, die 0,1 Gew.-% des Wirkstoffs enthält;
VI. eine innige Mischung aus 3 Gew.-Teilen der Verbindung 2.4 und 97 Gew.-Teilen feinteiligem Kaolin. Dieses Stäubemittel enthält 3 Gew.-% Wirkstoff;
VII. eine innige Mischung aus 30 Gew.-Teilen der Verbindung 2.7, 92 Gew.-Teilen pulverförmigem Kieselsäuregel und 8 Gew.-Teilen Paraffinöl, das auf die Oberfläche dieses Kieselsäuregels gesprüht wurde. Diese Aufbereitung gibt dem Wirkstoff eine gute Haftfähigkeit;
VIII. eine stabile wäßrige Dispersion aus 40 Gew.-Teilen der Verbindung 3.1, 10 Gew.-Teilen des Natriumsalzes eines Phenosulfonsäureharnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen der Verbindung 4.1, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehyd-Kondensates und 68 Gew.-Teilen eines paraffinischen Mineralöls;
X. eine in einer Hammermühle vermahlene Mischung aus 10 Gew.-Teilen der Verbindung 3.17, 4 Gew.-Teilen des Natriumsalzes der Diisobutylnaphthalin-α-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daβ die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 2,0 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus sinensis | Apfelsine, Orange |
| Coffea arabica (Coffea canephora, Coffea liberica) | Kaffee |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |

| Botanischer Name | Deutscher Name |
|---|---|
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus vulgaris | Buschbohne |
| Picea abies | Rotfichte |
| Pinus spp. | Kiefer |
| Pisum sativum | Gartenerbse |
| Prunus avium | Süßkirsche |
| Prunus persica | Pfirsich |
| Pyrus communis | Birne |
| Ribes sylvestre | Rote Johannisbeere |
| Ricinus communis | Rizinus |
| Saccharum officinarum | Zuckerrohr |
| Secale cereale | Roggen |
| Solanum tuberosum | Kartoffel |
| Sorghum bicolor (s. vulgare) | Mohrenhirse |
| Theobroma cacao | Kakaobaum |
| Trifolium pratense | Rotklee |
| Triticum aestivum | Weizen |
| Triticum durum | Hartweizen |
| Vicia faba | Pferdebohnen |
| Vitis vinifera | Weinrebe |
| Zea mays | Mais |

Zur Verbreiterung des Wirkungsspektrums und zur Erzielung synergistischer Effekte können die erfindungsgemäßen Verbindungen 1 mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Die im nachstehenden Synthesebeispiel I wiedergegebene Vorschrift wurde unter entsprechender Abwandlung der Ausgangsverbindung zur Gewinnung weiterer Verbindungen der Formel I benutzt; die erhaltenen Verbindungen sind in den nachfolgenden Tabellen mit physikalischen Angaben aufgeführt; Verbindungen ohne diese Angaben lassen sich aus den entsprechenden Stoffen in analoger Weise aufbauen. Sie lassen aufgrund ihrer nahen strukturellen Beziehungen zu den hergestellten und untersuchten Verbindungen eine gleichartige Wirkung erwarten.

### Synthesebeispiele

### I. Herstellung der Cyclohexenonoximether I

In 350 ml trockenes N-Methylpyrrolidon gab man nacheinander 18,5 g (0,11 mol) N-Hydroxyphthalimid und 31,4 g (0,11 mol) 1-Brom-[3-(4-Bromphenyl)]-prop-2-en und tropfte anschließend bei Raumtemperatur 12,1 g (0,12 mol) Triethylamin zu. Nach viertägigem Rühren bei 20°C wurde die Reaktionsmischung auf 1,5 l Eiswasser gegossen, abfiltriert und mit Wasser und Isopropanol nachgewaschen. Man erhielt 34,3 g (86,8 % d.Th.) N-[3-(4-Bromphenyl)-prop-2-enyloxi]-phthalimid mit dem Schmelzpunkt 161-162°C.

33,4 g (0,093 mol) N-[3(4-Bromphenyl)-prop-2-enyloxy]-phthalimid wurden portionsweise in 50 ml Ethanolamin eingetragen; die Temperatur stieg dabei bis auf 30°C an. Nach zweistündigem Rühren bei 60°C ließ man abkühlen und versetzte die Mischung mit 200 ml Dichlormethan. Es wurde mit Eiswasser ausgeschüttelt. Die organische Phase getrocknet und eingeengt und aus Petrolether kristallisiert. Dabei fielen 20,2 g (95,3 % d.Th.) 3-(4-Bromphenyl)-prop-2-enyloxiamin an. Schmp. 35-38°C.

3,0 g (0,011 mol) 2-Propionyl-5-(3-tetrahydrothiopyranyl)-cyclohexan-1,3-dion und 3,0 g (0,013 mol) 3-(4-Bromphenyl)-prop-2-enyloxiamin wurden in 100 ml Methanol bei 20°C 16 Stunden gerührt. Das dabei ausgefallene Reaktionsprodukt wurde bei 0°C abgesaugt und mit eiskaltem Methanol und Petrolether nachgewaschen. Nach dem Trocknen erhielt man 3,7 g (68,4 % d.Th.) 2[1-(3-(4-Bromphenyl)-prop-2-enyloximino)-propyl]-3-hydroxy-5-(3-tetrahydrothiopyranyl)-cyclohex-2-en-1-on mit dem Schmelzpunkt 97-99°C.

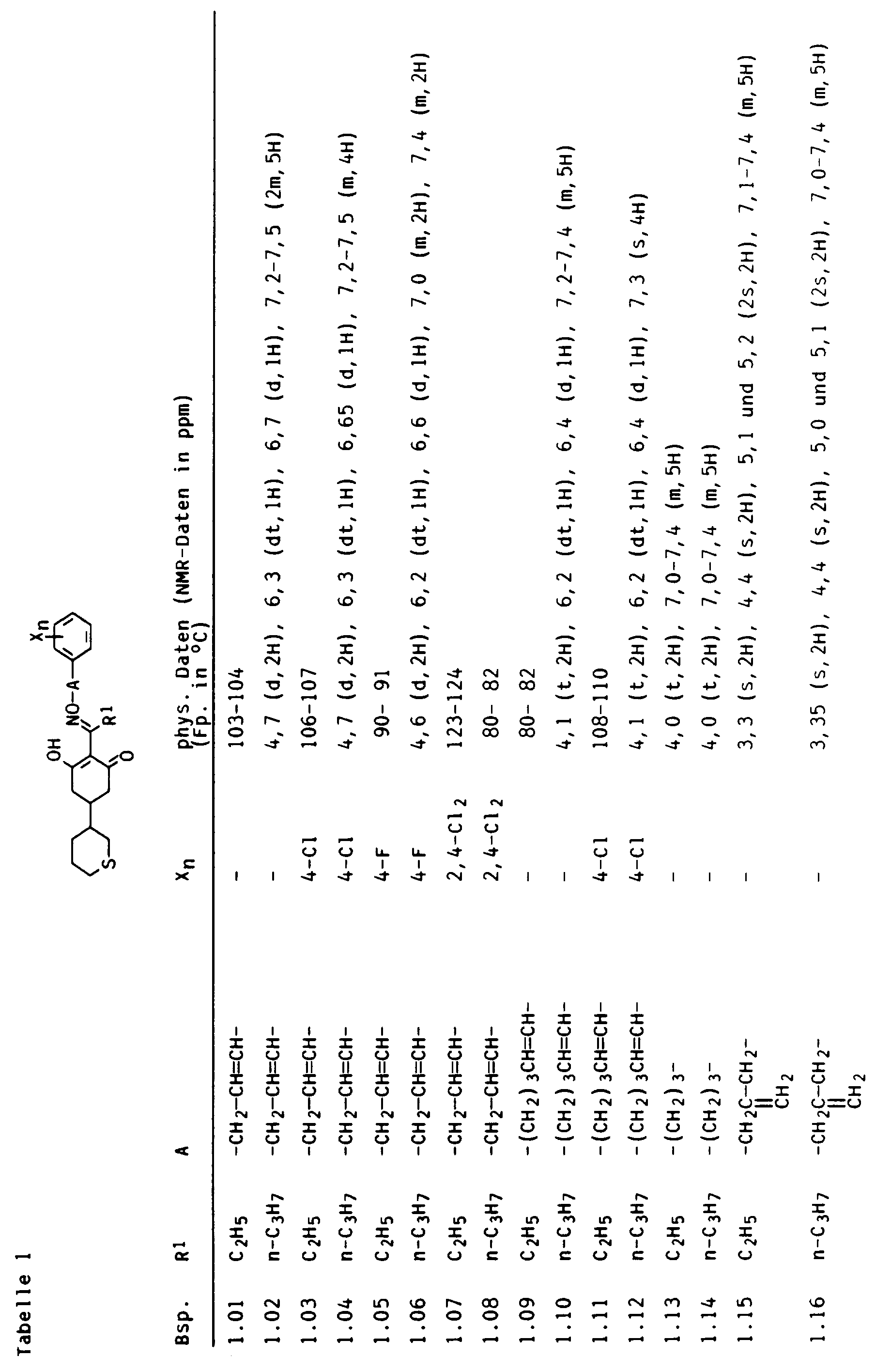

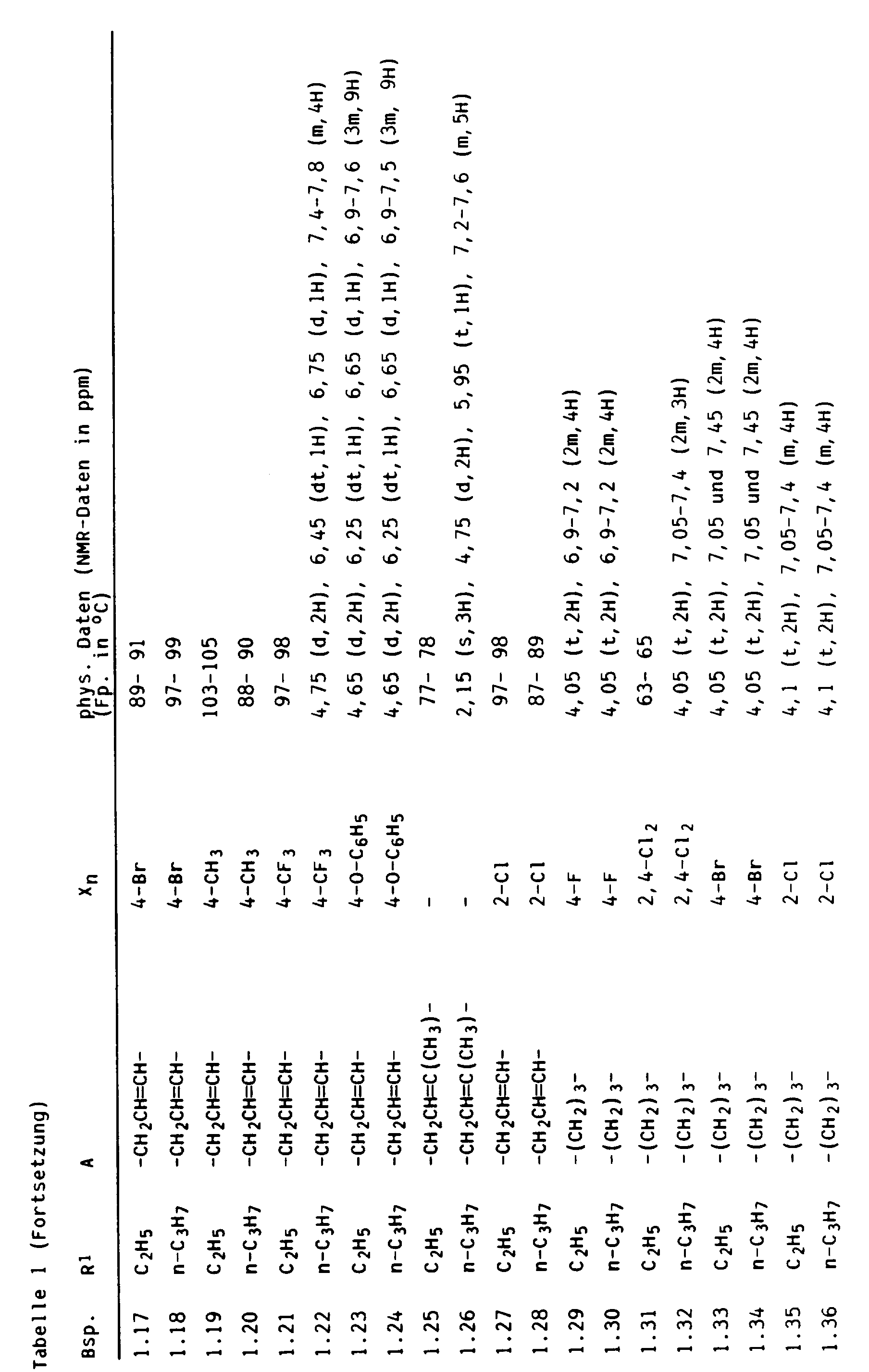

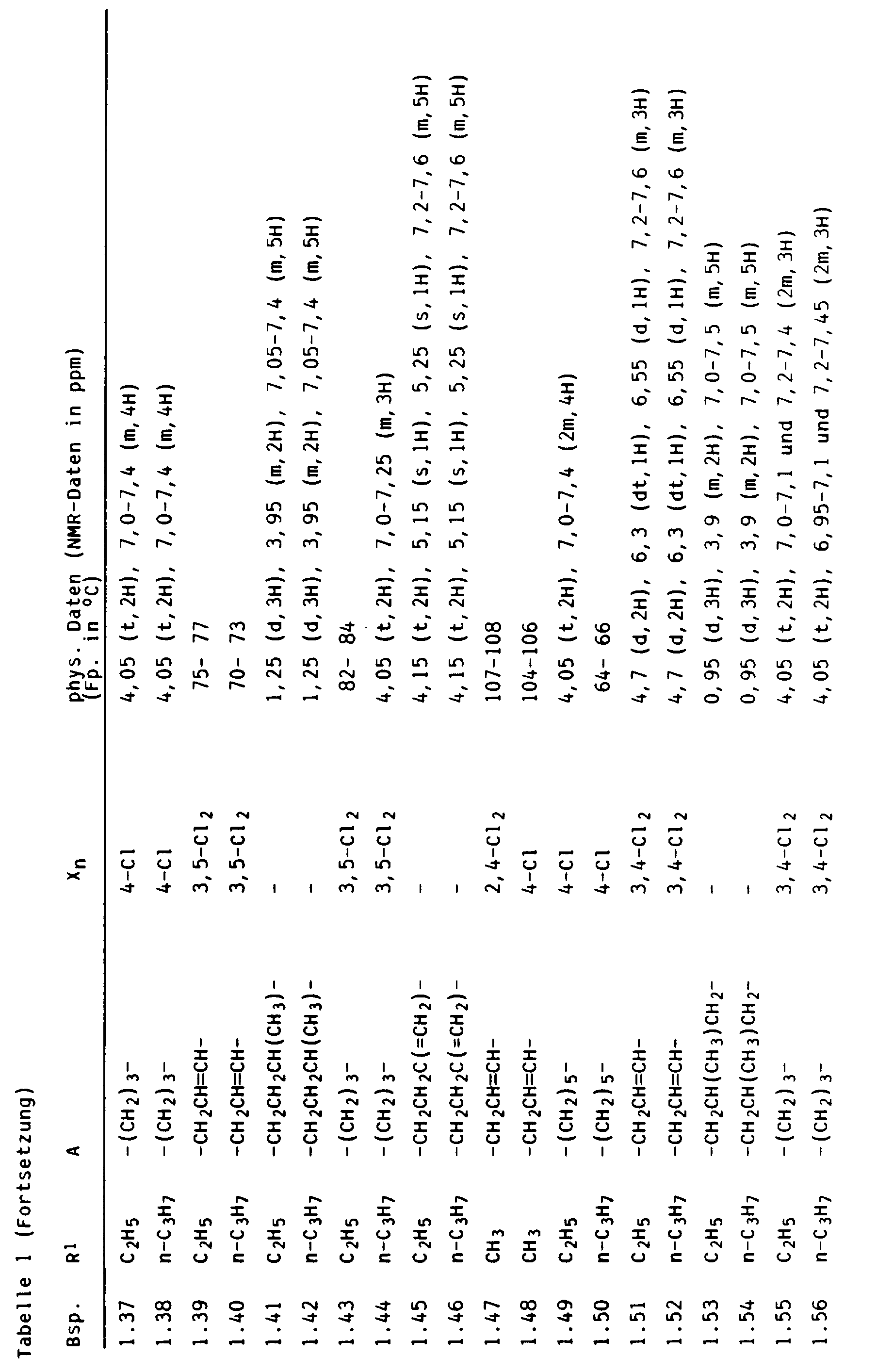

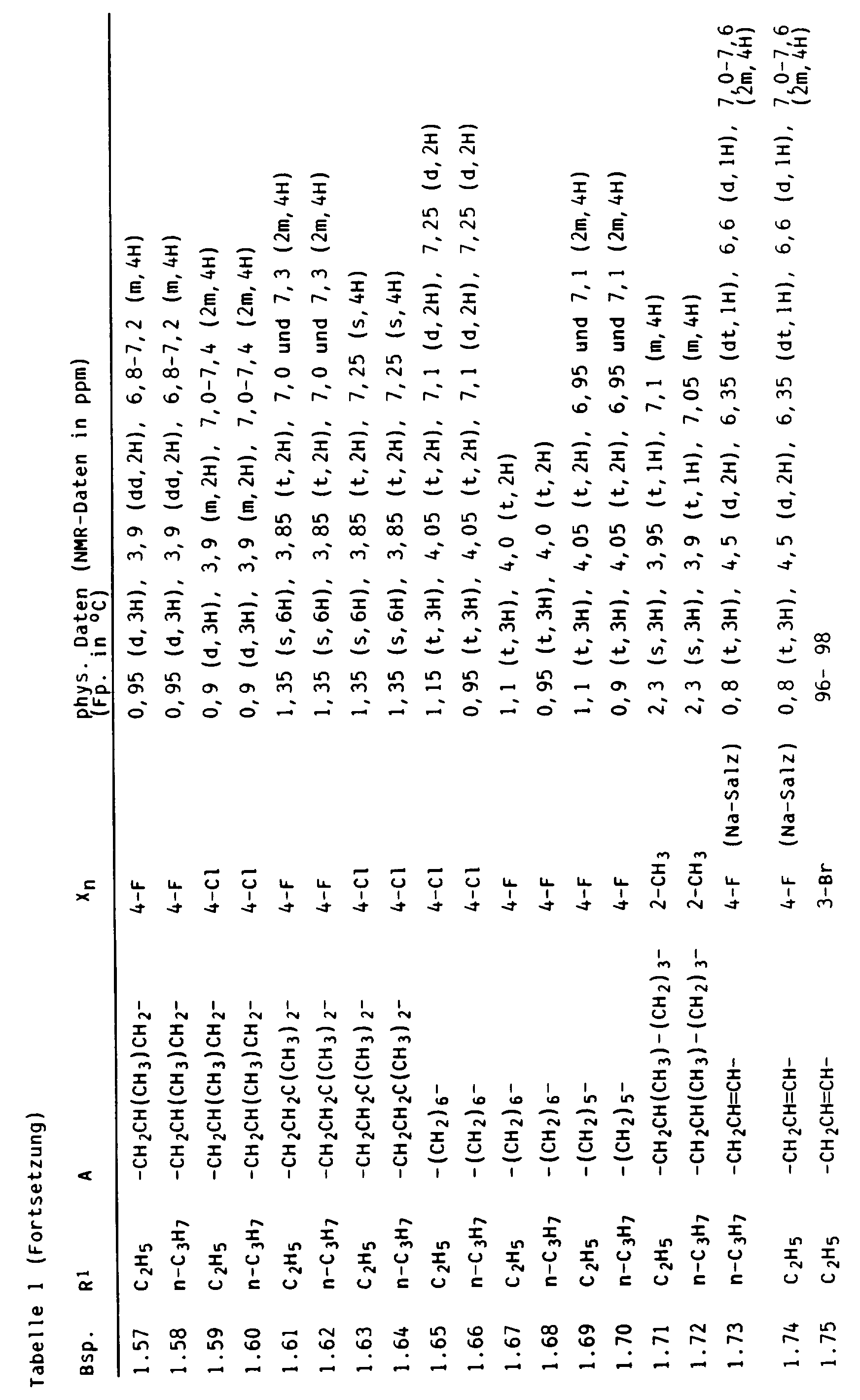

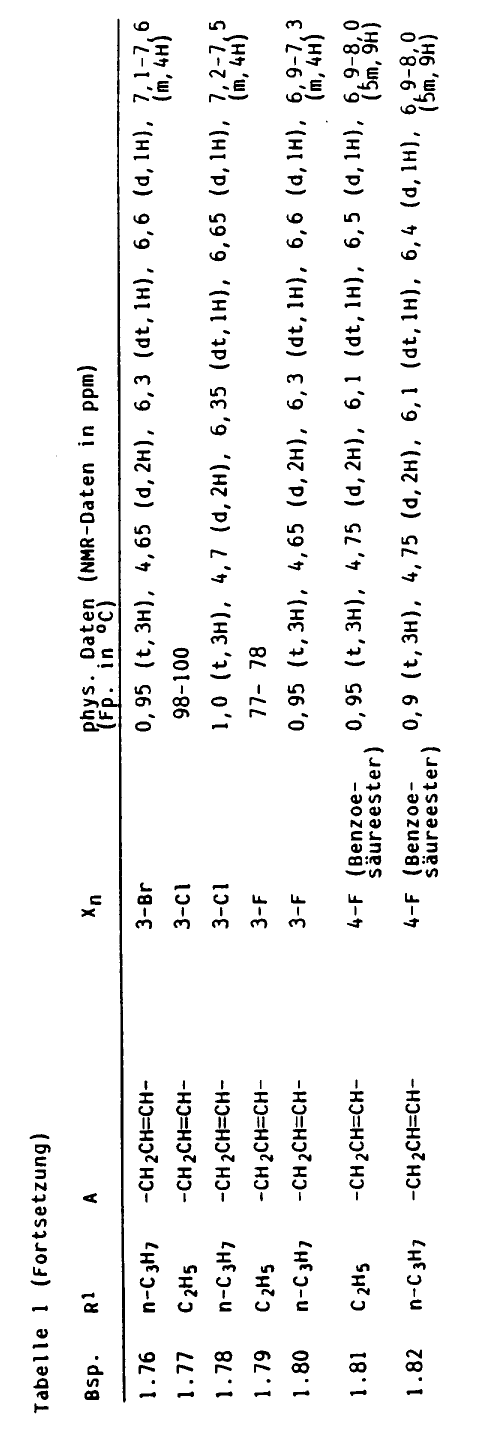

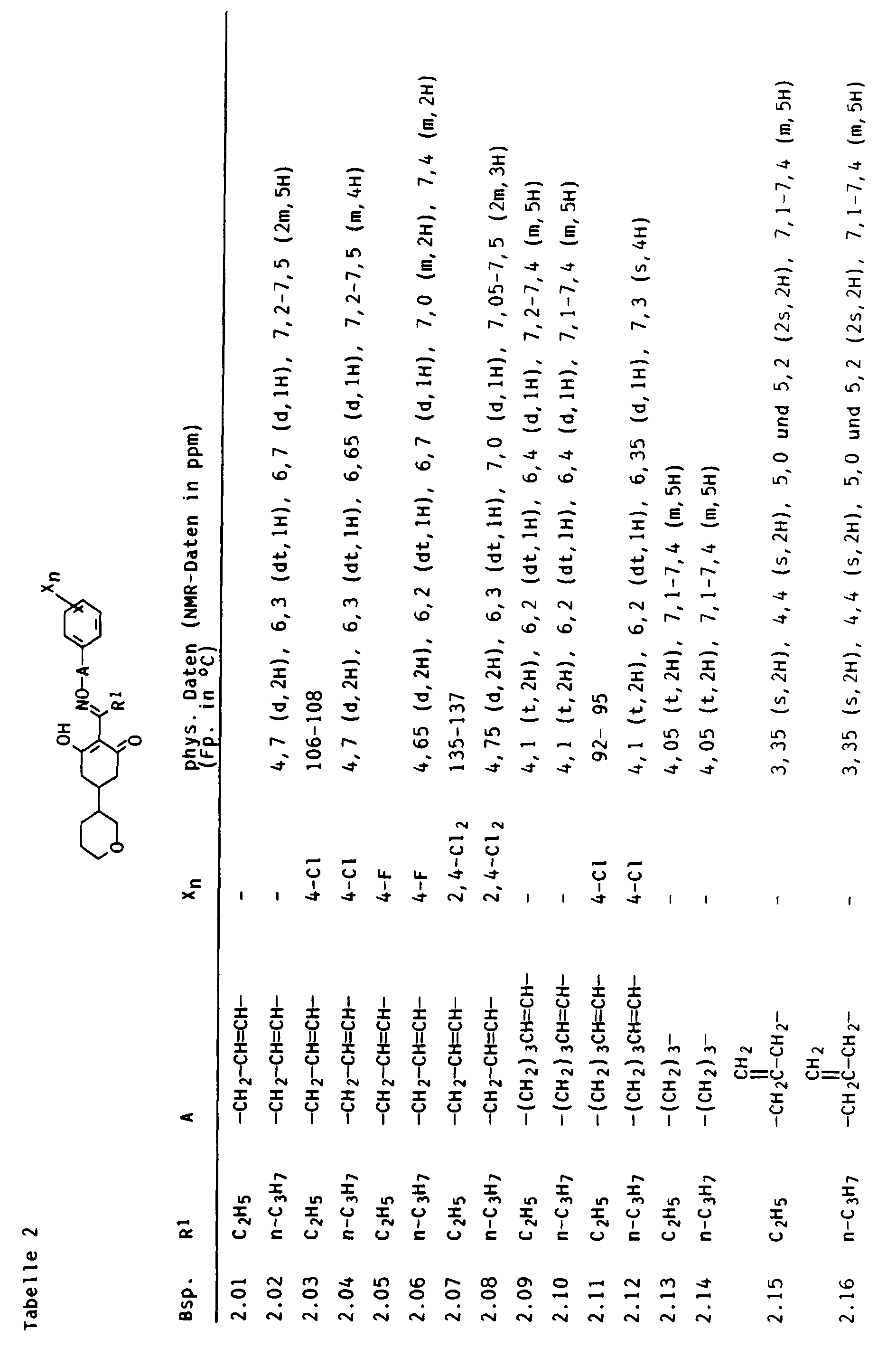

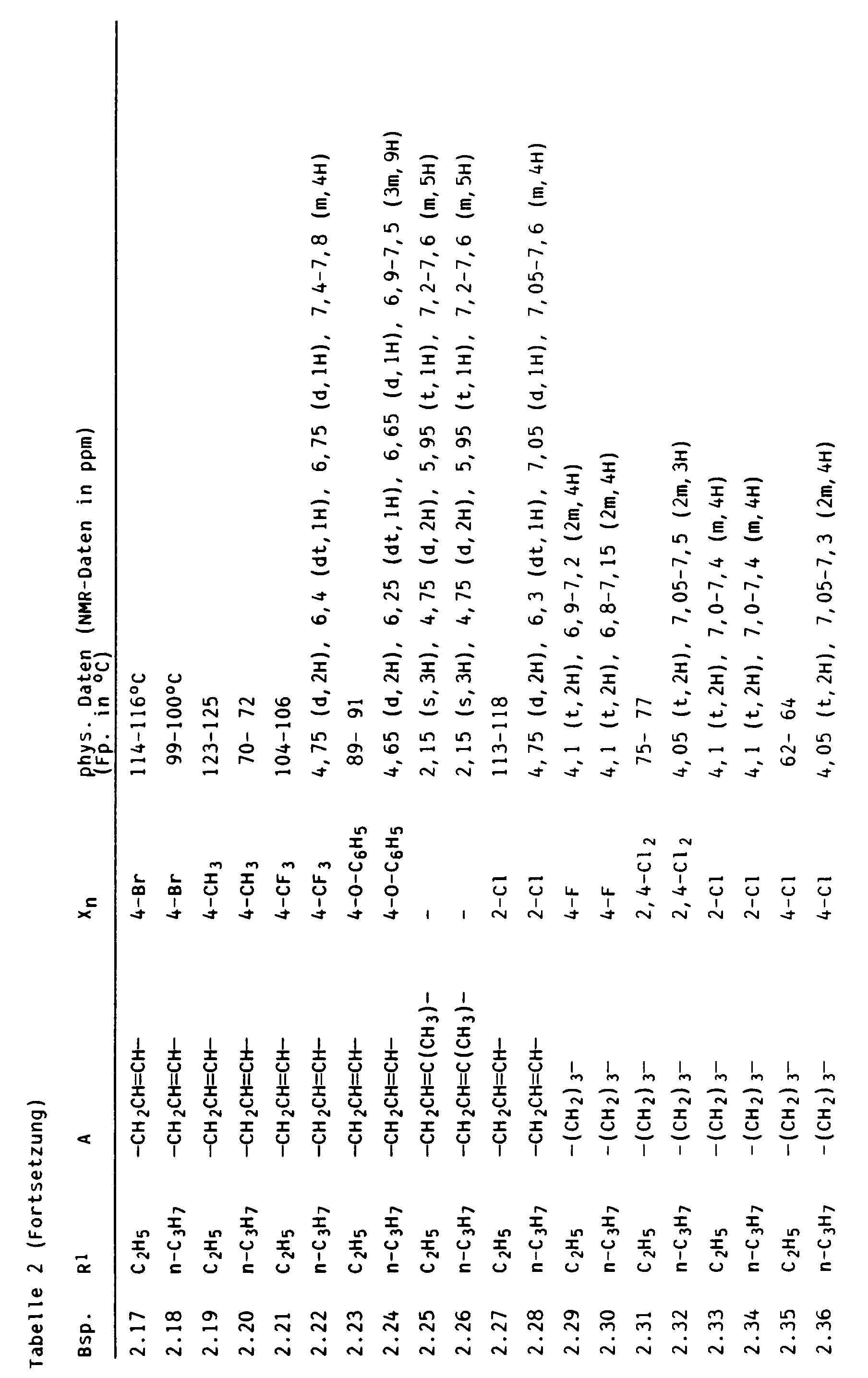

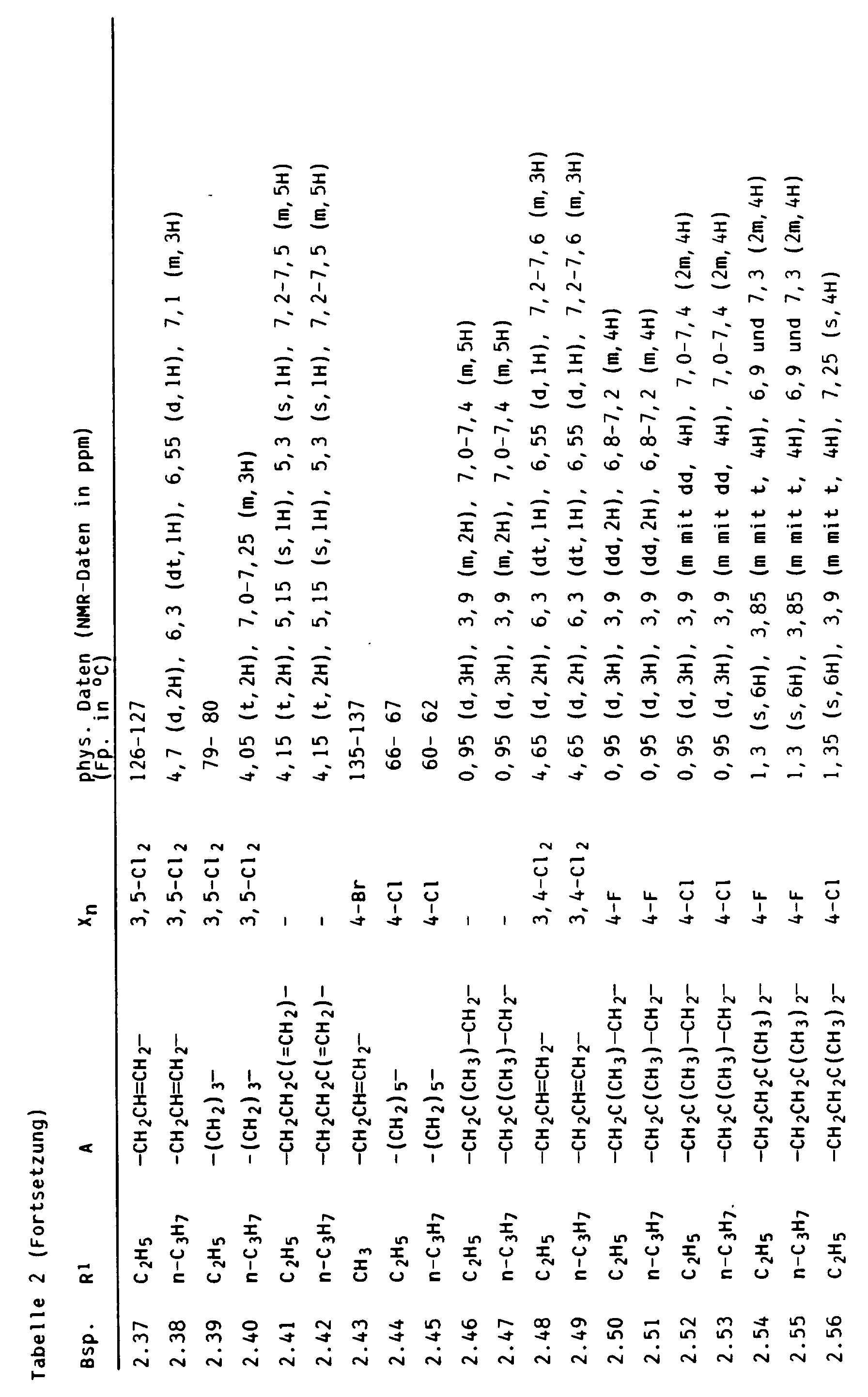

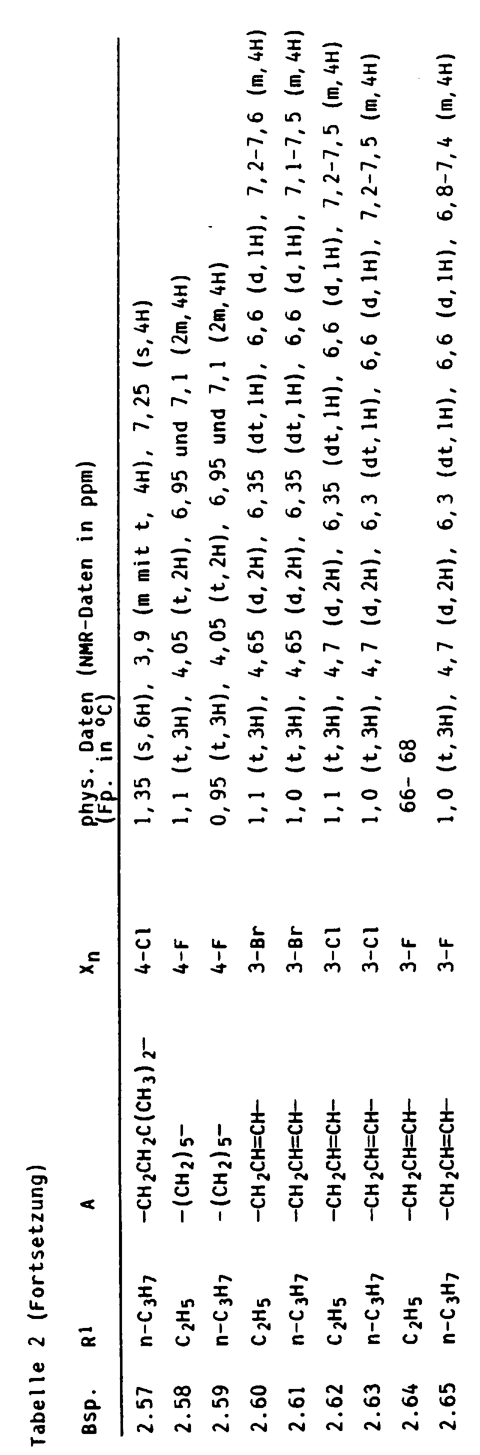

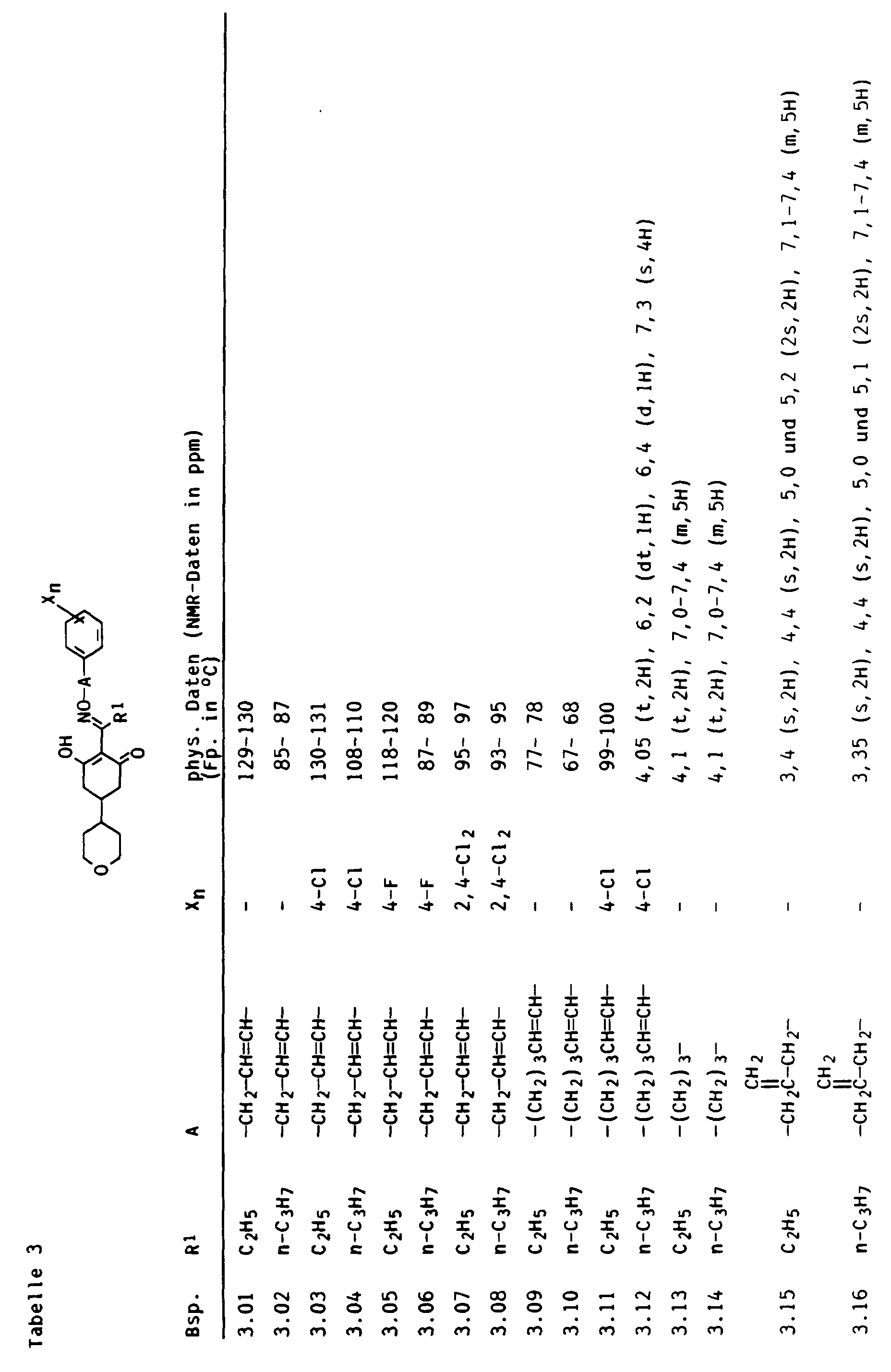

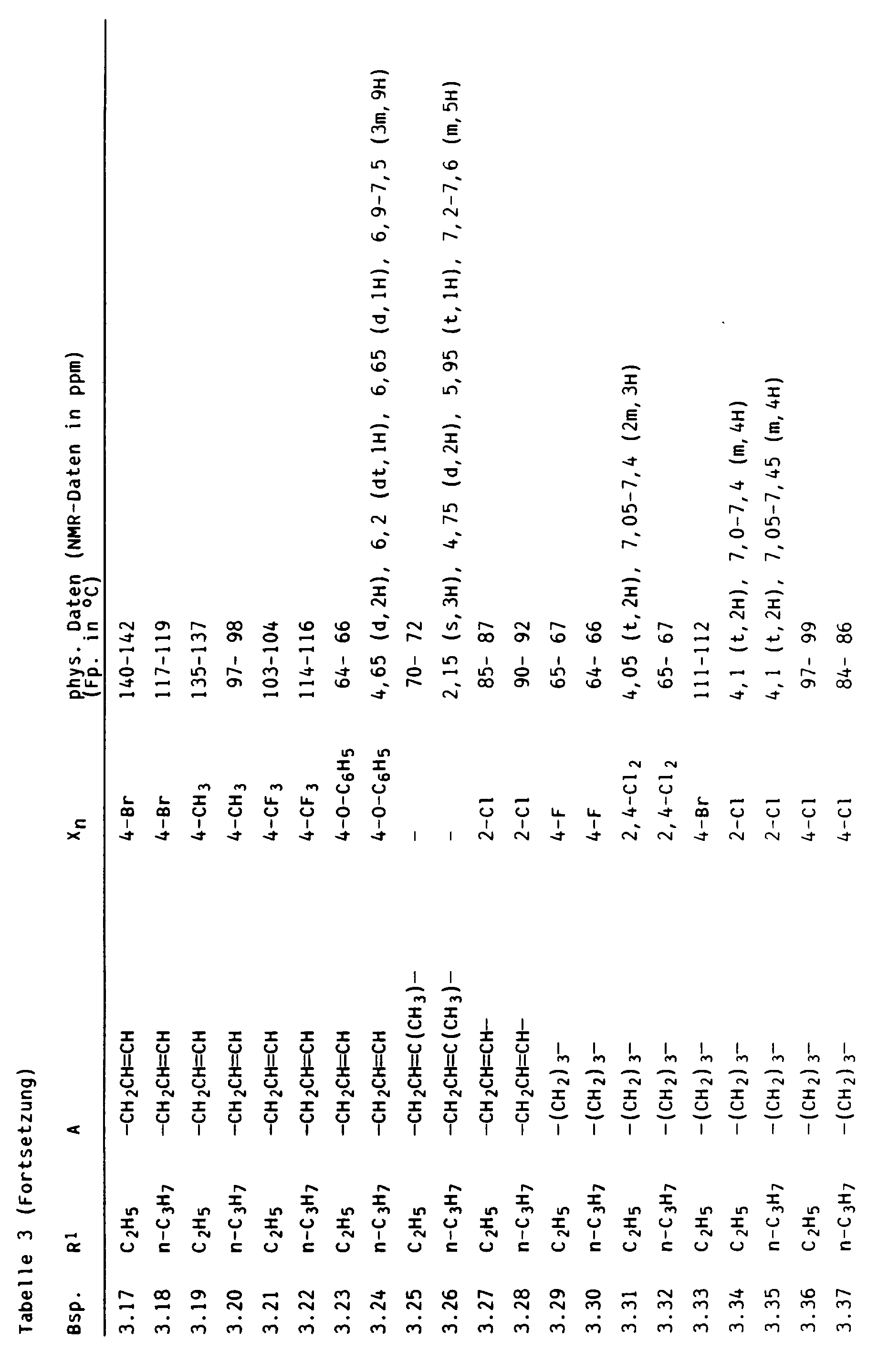

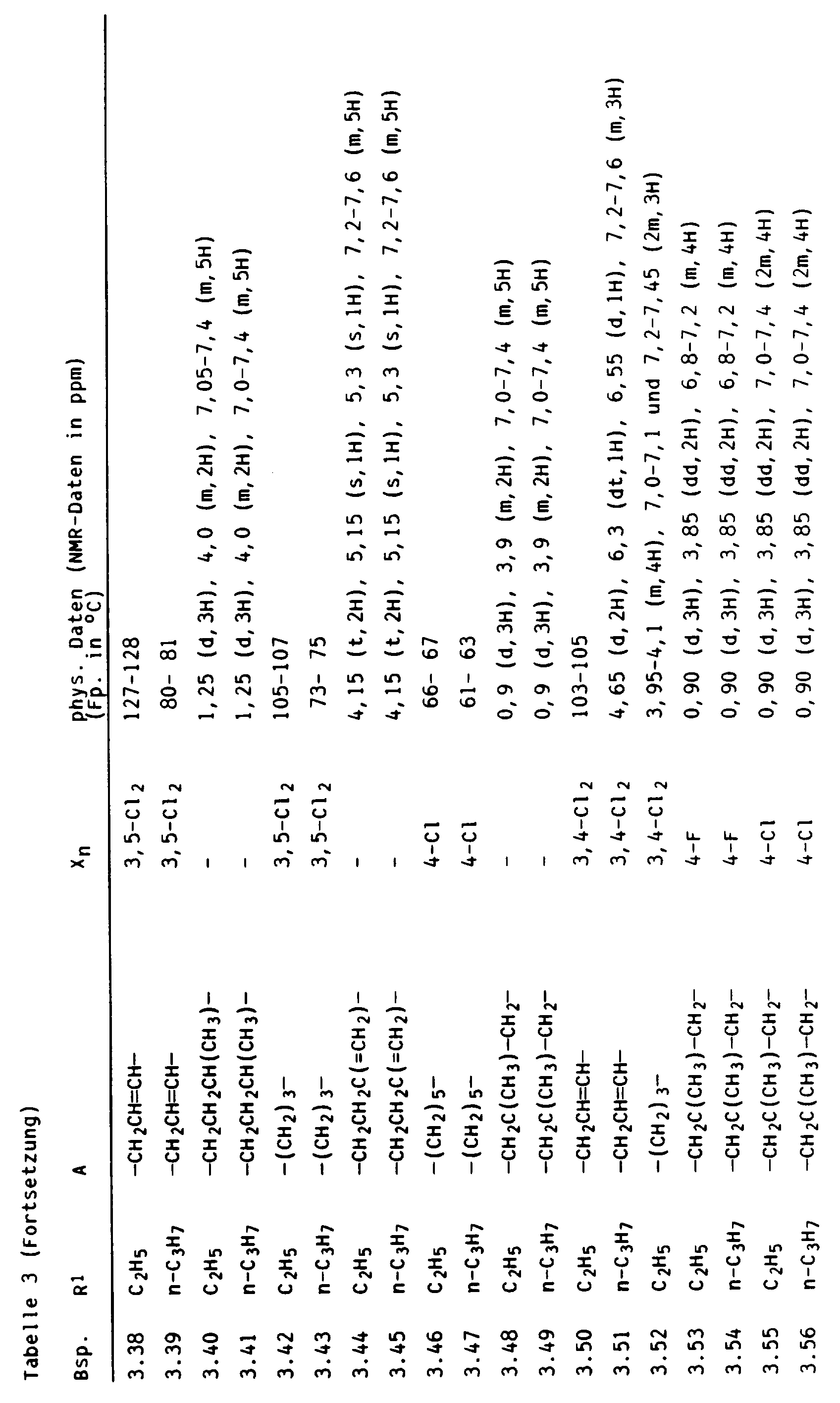

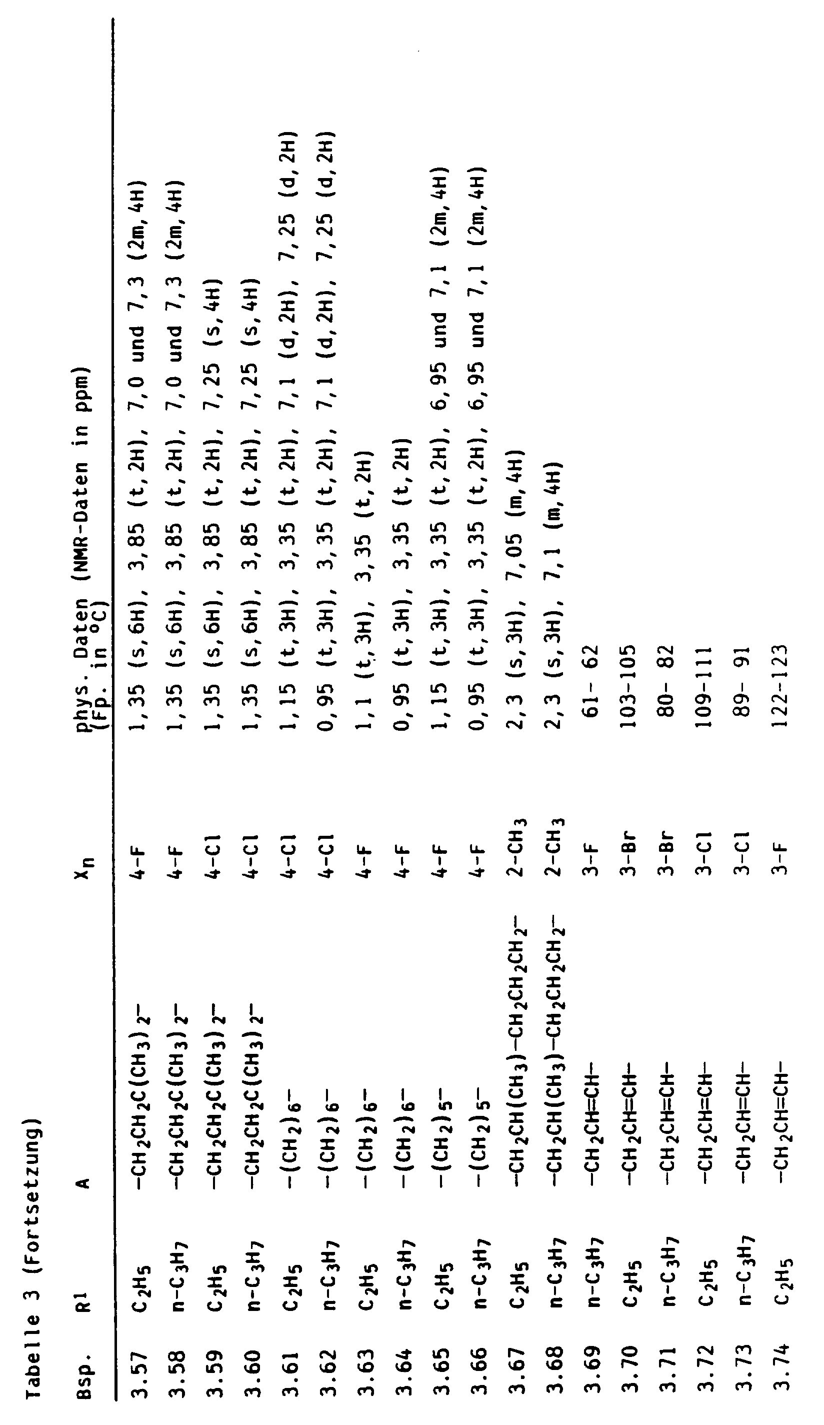

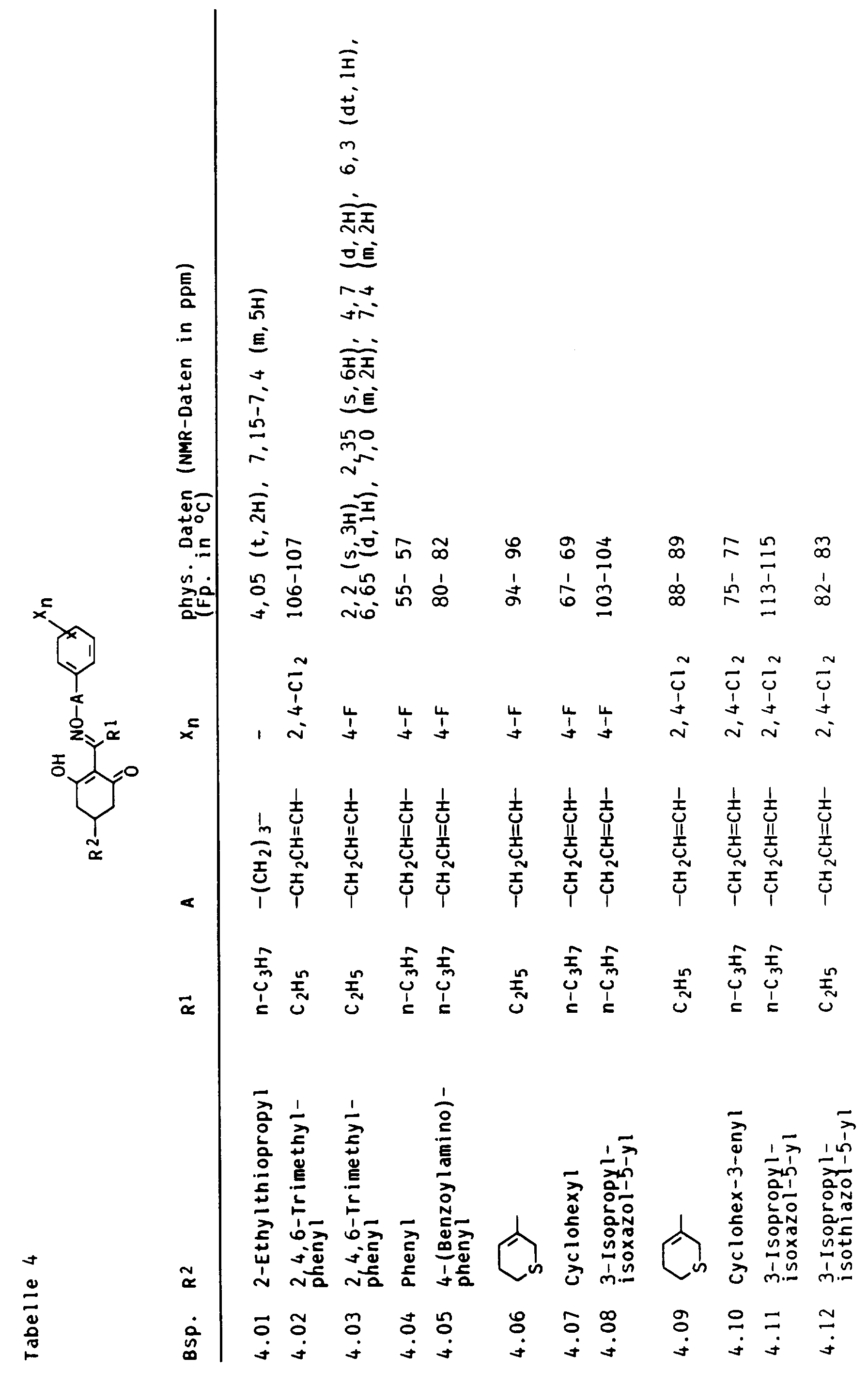

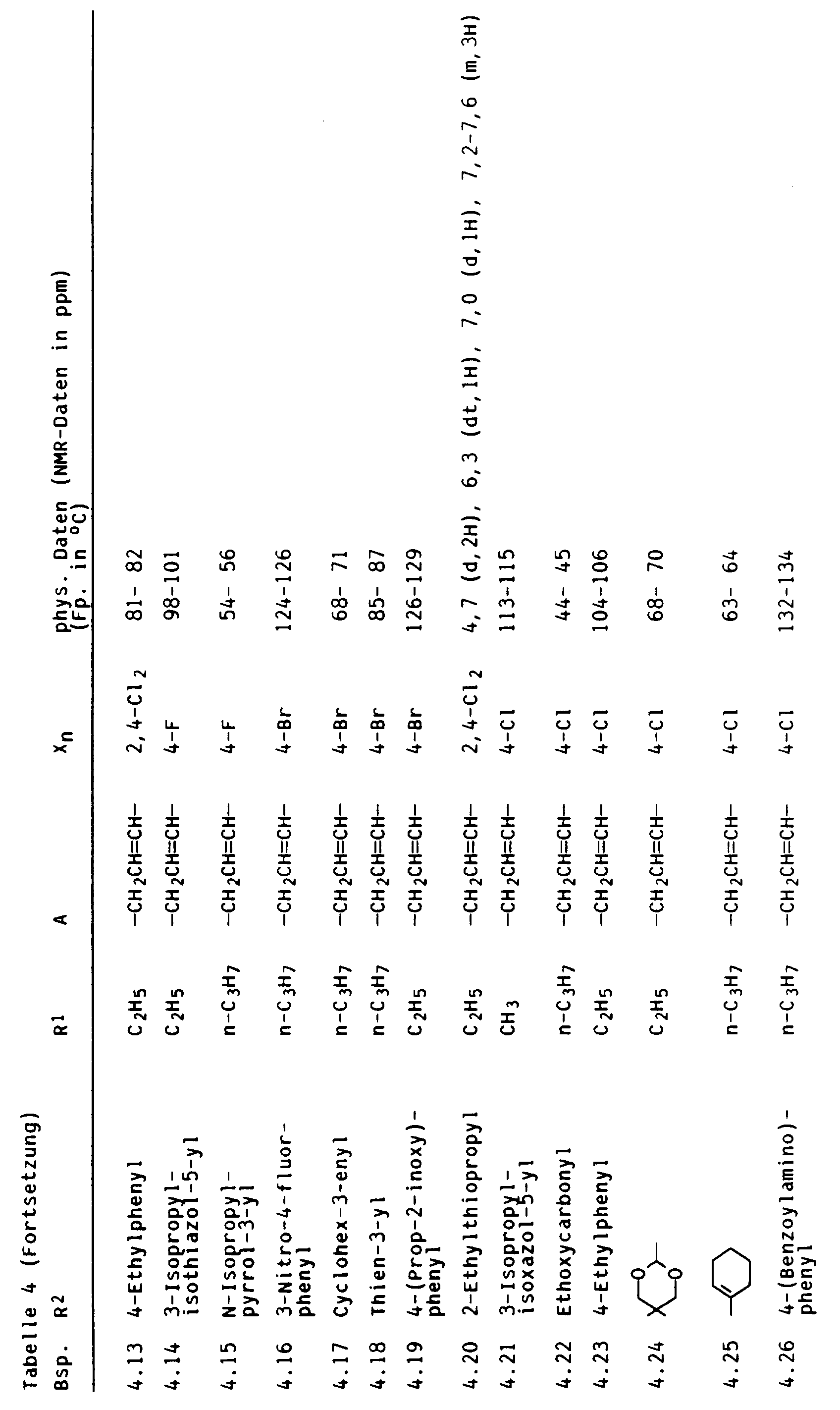

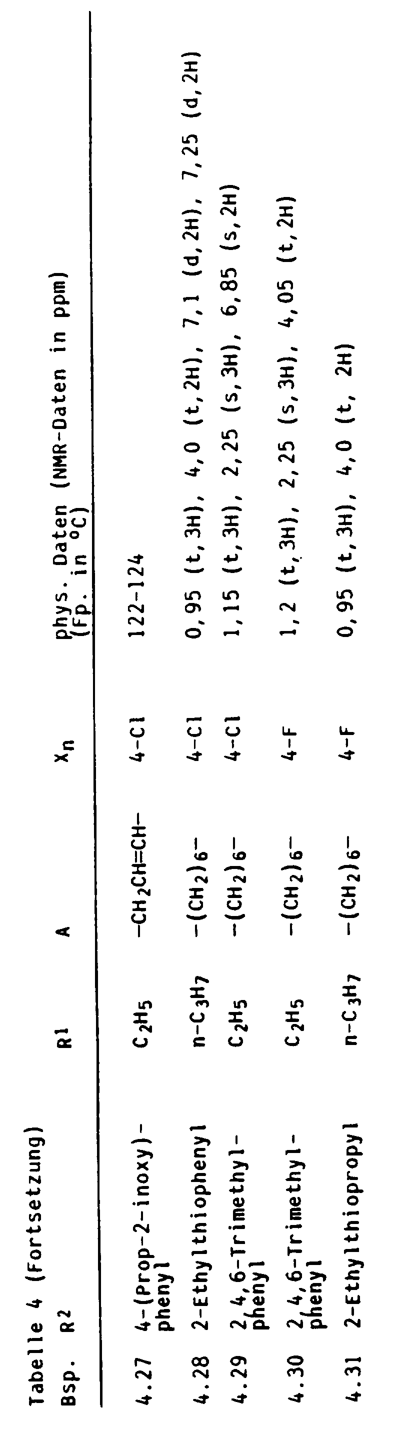

### II Herstellung der Hydroxylamine III

E-5-Aminooxy-1-phenyl-1-penten (Bsp. 5.14) 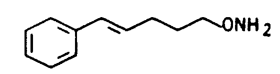 (E)-N-(5-phenyl-4-pentenyloxy)phthalimid

75,4 g (0,335 Mol) 5-Brom-1-phenyl-1-pentan (hergestellt durch Reduktion von E-5-Phenyl-4-pentansäureethylester mit Lithiumaluminiumhydrid und Behandlung des Alkohols mit Phosphortribromid) wurden zu einer Mischung, bestehend aus 340 ml N-Metthylpyrrolidin-2-on, 54,7 g (0,335 Mol) N-Hydroxyphthalimid und 5 g Kaliumiodid, innerhalb von 30 min bei 40°C getropft. Man rührte noch 4 Stunden bei 60°C, goß nach Abkühlen in 1,2 l Eiswasser, saugte den Festkörper ab, wusch diesen mit Wasser und kristallisierte aus Isopropanol um. Ausbeute: 91,4 g (89 %); Fp.: 93-94°C; 250-MHz-¹H-NMR (DMSO-d₆):
δ (ppm) =1,75-1,95 (m,2H), 2,3-2,5 (m,2H), 4,21 (t,2H), 6,25-6,55 (m,2H), 7,1-7,5 (m,5H), 7,87 (s,4H).

### E-5-Aminooxy-1-phenyl-1-penten

87,8 g (0,286 Mol) des oben hergestellten Phthalimidethers wurden in 130 ml Ethanolamin eingetragen. Nach 3 Stunden bei 60°C wurde die Mischung auf Raumtemperaur abgekühlt und in 200 ml Eiswasser gegossen. Man fügte 200 ml gesättigte Kochsalzlösung zu, wonach die Titelverbindung mit Dichlormethan (dreimal je 150 ml) extrahiert wurde. Die vereinigten organischen Phasen wurden mit gesättigter Kochsalzlösung (dreimal je 100 ml) gewaschen, getrocknet und unter reduziertem Druck eingeengt.
Ausbeute: 40 g (79 %);
250-MHz-¹H-NMR (DCl₃):
δ (ppm) =1,7-1,86 (m,2H), 2,2-2,37 (m,2H), 3,72 (t,2H), 5,35 (breites s,2H), 6,1-6,3 (m,1H), 6,4 (d,1H), 7,1-7,4 (m,5H).

In entsprechender Weise können die in Tabelle 5 wiedergegebenen Hydroxylamine III erhalten werden.

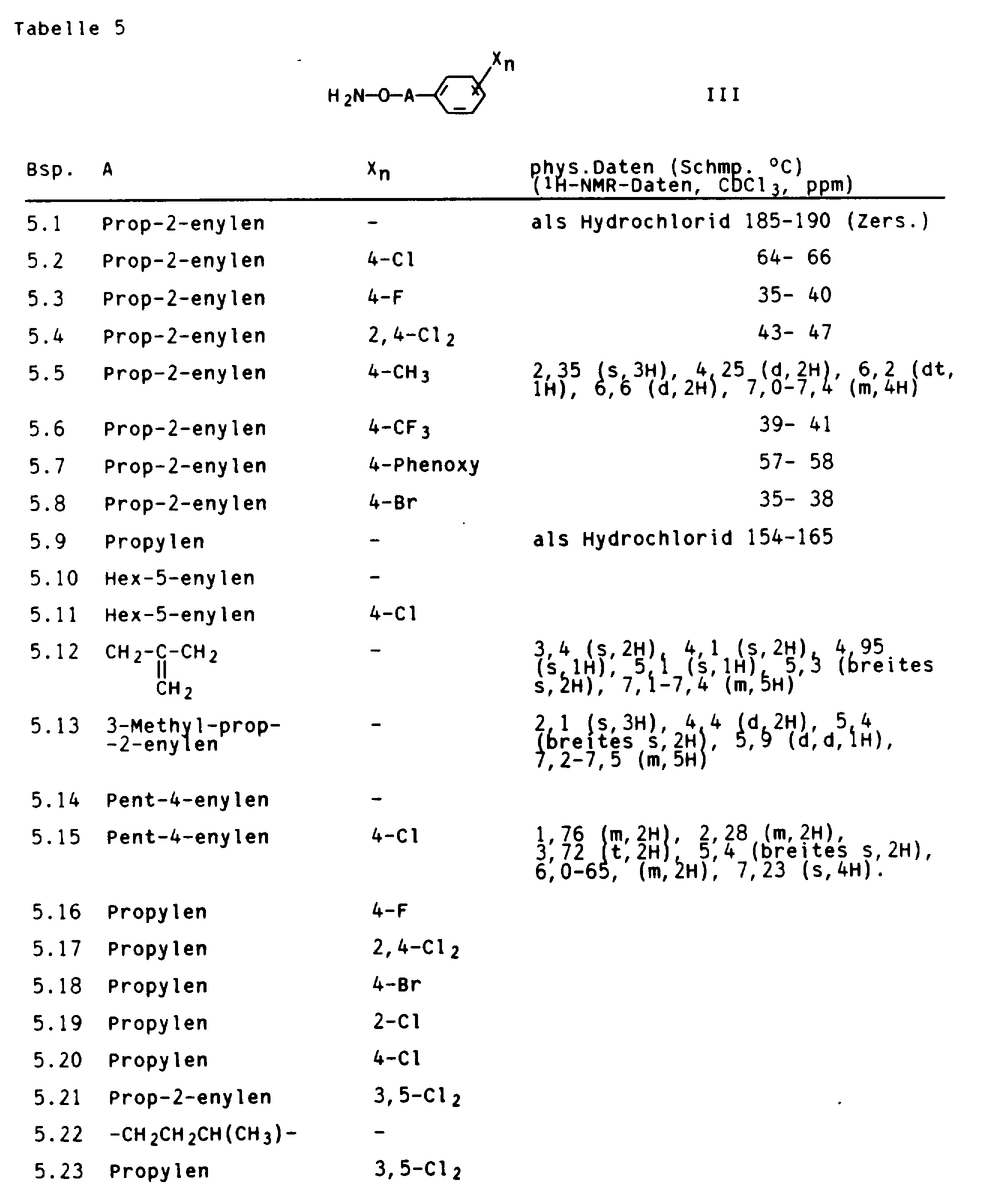

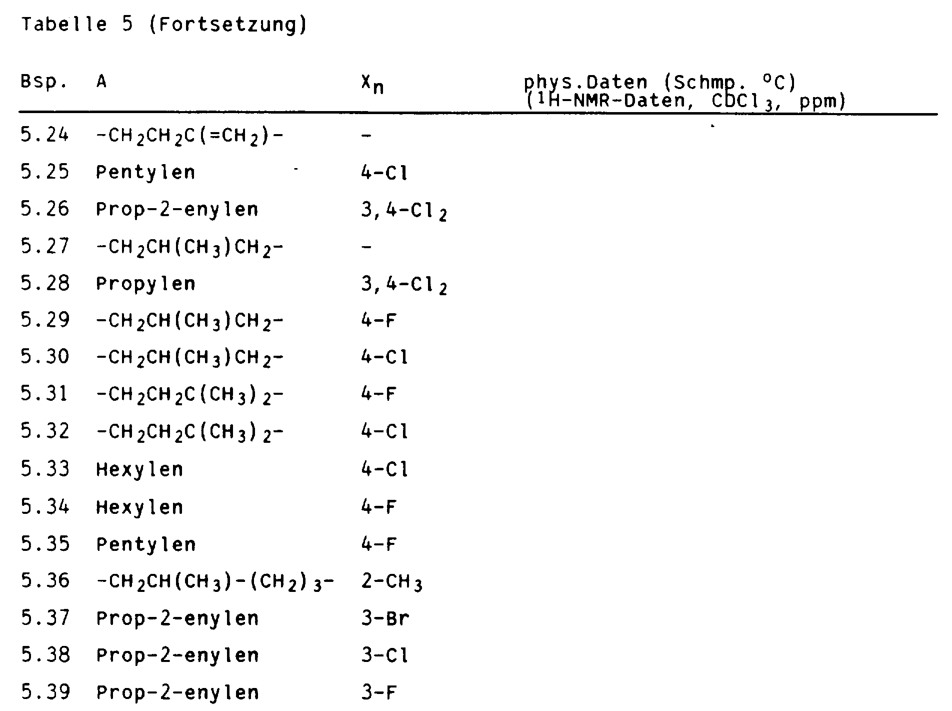

### Anwendungsbeispiele

Die herbizide Wirkung der Cyclohexenoximether der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 0,25 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Oryza sativa | Reis | rice |
| Echinochloa crus-galli | Hühnerhirse | barnyardgrass |
| Digitaria sanguinalis | Blutfingerhirse | - |

Mit 0,25 kg/ha a.S. im Nachauflaufverfahren eingesetzt, lassen sich mit dem Beispiel 1.1 unerwünschte grasartige Pflanzen sehr gut bekämpfen, bei gleichzeitiger Verträglichkeit an der Beispielkultur Reis.

## Patentansprüche (Patentansprüche für folgende(n) Vertragsstaat(en): AT, BE, CH, LI, DE, FR, GB, IT, NL)

1. Cyclohexenonoximether der allgemeinen Formel I 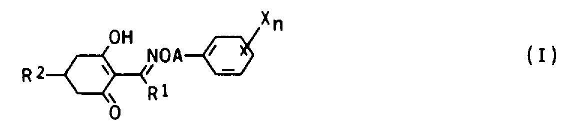 in der die Variablen folgende Bedeutung haben:
R¹ eine C₁-C₆-Alkylgruppe;
A eine C₃-, C₅-, C₆-Alkylen- oder C₃,C₅,C₆-Alkenylenkette, wobei diese Ketten ein bis drei C₁-C₃-Alkylgruppen oder eine Methylengruppe tragen können;
X Nitro, Cyano, Halogen, C₁-C₄-Alkyl, C₁-C₄-Alkoxy, Phenoxy-C₁-C₄-Alkylthio, C₁-C₄-Halogenalkyl, C₁-C₄-Halogenalkoxy, Carboxyl, C₁-C₄-Alkoxycarbonyl, Benzyloxycarbonyl und/oder Phenyl, wobei die aromatischen Substituenten noch einen bis drei Reste tragen können, ausgewählt aus einer Gruppe bestehend aus Nitro, Cyano, Halogen, C₁-C₄-Alkyl, C₁-C₄-Alkoxy, C₁-C₄-Alkylthio, C₁-C₄-Halogenalkyl, C₁-C₄-Halogenalkyloxy, Carboxyl, C₁-C₄-Alkoxycarbonyl- und Benzyloxycarbonyl, C₁-C₄-Alkoxy-C₁-C₆-alkyl;
n 0 bis 3 oder 1 bis 5 für den Fall, daß X Halogen bedeutet;
R² eine C₁-C₄-Alkoxy-C₁-C₆-alkyl- oder C₁-C₄-Alkylthio-C₁-C₆-alkylgruppe;
eine C₃-C₇-Cycloalkylgruppe oder eine C₅-C₇-Cycloalkenylgruppe, wobei diese Gruppen einen bis drei Reste tragen können, ausgewählt aus einer Gruppe bestehend aus C₁-C₄-Alkyl, C₁-C₄-Alkoxy, C₁-C₄-Alkylthio, C₁-C₄-Halogenalkyl, Hydroxy und Halogen;
ein 5-gliedriger gesättigter Heterocyclus der ein oder zwei Heteroatome enthält, ausgewählt aus einer Gruppe bestehend aus Sauerstoff und Schwefel, und der einen bis drei Reste tragen kann, ausgewählt aus einer Gruppe bestehend aus C₁-C₄-Alkyl, C₁-C₄-Alkoxy, C₁-C₄-Alkylthio und C₁-C₄-Halogenalkyl;
ein gesättigter oder partiell oder vollständig ungesättigter 6- oder 7-gliedriger Heterocyclus, enthaltend ein oder zwei Heteroatome, ausgewählt aus einer Gruppe bestehend aus Sauerstoff und Schwefel, wobei der Heterocyclus noch einen bis drei Reste tragen kann, ausgewählt aus einer Gruppe bestehend aus Hydroxy, Halogen, C₁-C₄-Alkyl, C₁-C₄-Alkoxy, C₁-C₄-Alkylthio und C₁-C₄-Halogenalkyl,
ein 5-gliedriger Heteroaromat, enthaltend ein bis zwei Stickstoffatome und ein Sauerstoffatom oder ein Schwefelatom, wobei dieser Ring noch einen bis drei Reste tragen kann, ausgewählt aus einer Gruppe bestehend aus Halogen, Cyano, C₁-C₄-Alkyl, C₁-C₄-Alkoxy. C₁-C₄-Alkylthio, C₁-C₄-Halogenalkyl, C₂-C₆-Alkenyl, C₂-C₆-Alkenyloxy, C₂-C₆-Halogenalkenyl und C₁-C₄-Alkoxy-C₁-C₄-alkyl,
die Phenyl- oder die Pyridylgruppe, wobei diese Gruppen noch einen bis drei Reste tragen können, ausgewählt aus einer Gruppe bestehend aus Halogen, Nitro, Cyano, C₁-C₄-Alkyl, C₁-C₄-Alkoxy, C₁-C₄-Alkylthio, C₁-C₄-Halogenalkyl, C₃-C₆-Alkenyloxy, C₃-C₆-Alkinyloxy und -NR³R⁴, worin
R³ Wasserstoff, eine C₁-C₄-Alkylgruppe, eine C₃-C₆-Alkenylgruppe oder eine C₃-C₆-Alkinylgruppe und
R⁴ Wasserstoff, eine C₁-C₄-Alkylgruppe, eine C₃-C₆-Alkenylgruppe, eine C₃-C₆-Alkinylgruppe, eine C₁-C₆-Acylgruppe oder ein Benzoylrest, wobei der aromatische Ring noch einen bis drei Reste tragen kann, ausgewählt aus einer Gruppe bestehend aus Nitro, Cyano, Halogen, C₁-C₄-Alkyl, C₁-C₄-Alkoxy, C₁-C₄-Alkylthio und C₁-C₄-Halogenalkyl
bedeuten,
sowie ihre landwirtschaftlich nutzbaren Salze und Ester von C₁-C₁₀-Carbonsäuren und anorganischen Säuren.

2. Verfahren zur Herstellung von Verbindungen der Formel I gemäß Anspruch 1, dadurch gekennzeichnet, daß man ein entsprechendes Cyclohexenon der Formel II 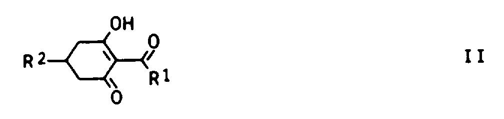 in an sich bekannter Weise in einem inerten organischen Lösungsmittel mit einem Hydroxylamin der Formel III 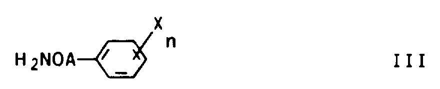 oder einem Salz des entsprechenden Hydroxylamins umsetzt.

3. Herbizides Mittel, enthaltend inerte Zusatzstoffe und eine herbizid wirksame Menge mindestens einer Verbindung der Formel I gemäß Anspruch 1.

4. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Cyclohexenonderivates der Formel I gemäß Anspruch 1 behandelt.

5. Hydroxylamine der Formel III 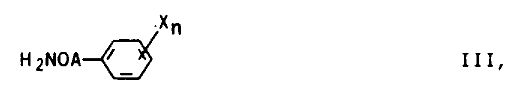 in der A und Xₙ folgende Bedeutung haben:
A eine C₃-, C₅- oder C₆-Alkylen- oder eine C₃- ,C₅- oder C₆-Alkenylenkette, wobei diese Ketten ein bis drei C₁-C₃-Alkylgruppen oder eine Methylengruppe tragen können;
X Nitro, Cyano, Halogen, C₁-C₄-Alkyl, C₁-C₄-Alkoxy, Phenoxy, C₁-C₄-Alkylthio, C₁-C₄-Halogenalkyl, C₁-C₄-Halogenalkoxy, Carboxyl, C₁-C₄-Alkoxycarbonyl, Benzyloxycarbonyl und/oder Phenyl, wobei die aromatischen Reste ein bis drei der folgenden Substituenten tragen können: Nitro, Cyano, Halogen, C₁-C₄-Alkyl, C₁-C₄-Alkoxy, C₁-C₄-Alkylthio, C₁-C₄-Halogenalkyl, C₁-C₄-Halogenalkyloxy, Carboxyl, C₁-C₄-Alkoxycarbonyl und/oder Benzyloxycarbonyl, C₁-C₄-Alkoxy-C₁-C₆-alkyl;
n 0 bis 3 oder 1 bis 5 für den Fall, daß X Halogen bedeutet.

6. Verfahren zur Herstellung der Hydroxylamine der allgemeinen Formel III gemäß Anspruch 5, dadurch gekennzeichnet, daß man Verbindungen der Formel VI 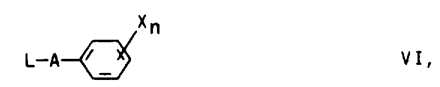 in der L für eine nukleophil verdrängbare Abgangsgruppe steht, umsetzt mit einem Hydroxyimid der Formel VII 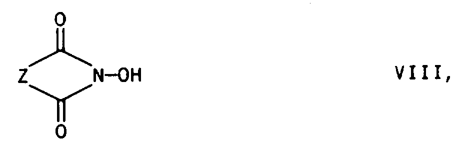 in der Z für einen Phenylen-, Naphthylen, Pyridinylen-, Cyclopentylen-, Cyclohexylen-, Cyclohexenylen-, C₂- bis C₄-Alkenylen- oder C₁- bis C₄-Alkylenrest steht, wobei diese Reste Z unsubstituiert sind oder 1, 2, 3 oder 4 Halogen-, C₁- bis C₄-Alkyl- und/oder C₁- bis C₄-Halogenalkyl-Substituenten tragen können, in Gegenwart eines Lösungsmittels und einer Base bei Temperaturen von 0 bis 140°C umsetzt und aus den erhaltenen Imidethern die Hydroxylamine III mittels Basen oder Säuren freisetzt.

## Patentansprüche (Patentansprüche für folgende(n) Vertragsstaat(en): ES)

1. Verfahren zur Herstellung von Cyclohexenonoximethern der allgemeinen Formel I 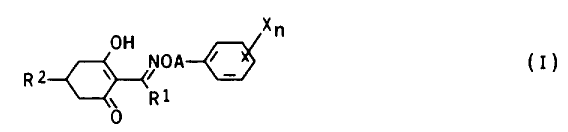 in der die Variablen folgende Bedeutung haben:
R¹ eine C₁-C₆-Alkylgruppe;
A eine C₃-, C₅-, C₆-Alkylen- oder C₃,C₅,C₆-Alkenylenkette, wobei diese Ketten ein bis drei C₁-C₃-Alkylgruppen oder eine Methylengruppe tragen können;
X Nitro, Cyano, Halogen, C₁-C₄-Alkyl, C₁-C₄-Alkoxy, Phenoxy-C₁-C₄-Alkylthio, C₁-C₄-Halogenalkyl, C₁-C₄-Halogenalkoxy, Carboxyl, C₁-C₄-Alkoxycarbonyl, Benzyloxycarbonyl und/oder Phenyl, wobei die aromatischen Substituenten noch einen bis drei Reste tragen können, ausgewählt aus einer Gruppe bestehend aus Nitro, Cyano, Halogen, C₁-C₄-Alkyl, C₁-C₄-Alkoxy, C₁-C₄-Alkylthio, C₁-C₄-Halogenalkyl, C₁-C₄-Halogenalkyloxy, Carboxyl, C₁-C₄-Alkoxycarbonyl- und Benzyloxycarbonyl, C₁-C₄-Alkoxy-C₁-C₆-alkyl;
n 0 bis 3 oder 1 bis 5 für den Fall, daß X Halogen bedeutet;
R² eine C₁-C₄-Alkoxy-C₁-C₆-alkyl- oder C₁-C₄-Alkylthio-C₁-C₆-alkylgruppe;
eine C₃-C₇-Cycloalkylgruppe oder eine C₅-C₇-Cycloalkenylgruppe, wobei diese Gruppen einen bis drei Reste tragen können, ausgewählt aus einer Gruppe bestehend aus C₁-C₄-Alkyl, C₁-C₄-Alkoxy, C₁-C₄-Alkylthio, C₁-C₄-Halogenalkyl, Hydroxy und Halogen;
ein 5-gliedriger gesättigter Heterocyclus der ein oder zwei Heteroatome enthält, ausgewählt aus einer Gruppe bestehend aus Sauerstoff und Schwefel, und der einen bis drei Reste tragen kann, ausgewählt aus einer Gruppe bestehend aus C₁-C₄-Alkyl, C₁-C₄-Alkoxy, C₁-C₄-Alkylthio und C₁-C₄-Halogenalkyl;
ein gesättigter oder partiell oder vollständig ungesättigter 6- oder 7-gliedriger Heterocyclus, enthaltend ein oder zwei Heteroatome, ausgewählt aus einer Gruppe bestehend aus Sauerstoff und Schwefel, wobei der Heterocyclus noch einen bis drei Reste tragen kann, ausgewählt aus einer Gruppe bestehend aus Hydroxy, Halogen, C₁-C₄-Alkyl, C₁-C₄-Alkoxy, C₁-C₄-Alkylthio und C₁-C₄-Halogenalkyl,
ein 5-gliedriger Heteroaromat, enthaltend ein bis zwei Stickstoffatome und ein Sauerstoffatom oder ein Schwefelatom, wobei dieser Ring noch einen bis drei Reste tragen kann, ausgewählt aus einer Gruppe bestehend aus Halogen, Cyano, C₁-C₄-Alkyl, C₁-C₄-Alkoxy. C₁-C₄-Alkylthio, C₁-C₄-Halogenalkyl, C₂-C₆-Alkenyl, C₂-C₆-Alkenyloxy, C₂-C₆-Halogenalkenyl und C₁-C₄-Alkoxy-C₁-C₄-alkyl,
die Phenyl- oder die Pyridylgruppe, wobei diese Gruppen noch einen bis drei Reste tragen können, ausgewählt aus einer Gruppe bestehend aus Halogen, Nitro, Cyano, C₁-C₄-Alkyl, C₁-C₄-Alkoxy, C₁-C₄-Alkylthio, C₁-C₄-Halogenalkyl, C₃-C₆-Alkenyloxy, C₃-C₆-Alkinyloxy und -NR³R⁴, worin
R³ Wasserstoff, eine C₁-C₄-Alkylgruppe, eine C₃-C₆-Alkenylgruppe oder eine C₃-C₆-Alkinylgruppe und
R⁴ Wasserstoff, eine C₁-C₄-Alkylgruppe, eine C₃-C₆-Alkenylgruppe, eine C₃-C₆-Alkinylgruppe, eine C₁-C₆-Acylgruppe oder ein Benzoylrest, wobei der aromatische Ring noch einen bis drei Reste tragen kann, ausgewählt aus einer Gruppe bestehend aus Nitro, Cyano, Halogen, C₁-C₄-Alkyl, C₁-C₄-Alkoxy, C₁-C₄-Alkylthio und C₁-C₄-Halogenalkyl
bedeuten,
sowie ihre landwirtschaftlich nutzbaren Salze und Ester von C₁-C₁₀-Carbonsäuren und anorganischen Säuren, dadurch gekennzeichnet, daß man ein entsprechendes Cyclohexenon der Formel II 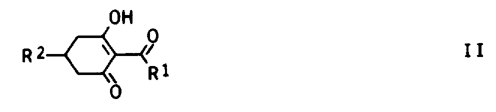 in an sich bekannter Weise in einem inerten organischen Lösungsmittel mit einem Hydroxylamin der Formel III 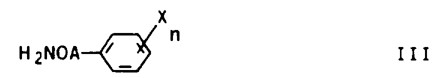 oder einem Salz des entsprechenden Hydroxylamins umsetzt.

2. Herbizides Mittel, enthaltend inerte Zusatzstoffe und eine herbizid wirksame Menge mindestens einer Verbindung der Formel I gemäß Anspruch 1.

3. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Cyclohexenonderivates der Formel I gemäß Anspruch 1 behandelt.

4. Verfahren zur Herstellung der Hydroxylamine der allgemeinen Formel III 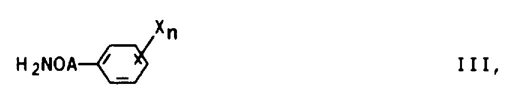 in der A und Xₙ folgende Bedeutung haben:
A eine C₃-, C₅- oder C₆-Alkylen- oder eine C₃- ,C₅- oder C₆-Alkenylenkette, wobei diese Ketten ein bis drei C₁-C₃-Alkylgruppen oder eine Methylengruppe tragen können;
X Nitro, Cyano, Halogen, C₁-C₄-Alkyl, C₁-C₄-Alkoxy, Phenoxy, C₁-C₄-Alkylthio, C₁-C₄-Halogenalkyl, C₁-C₄-Halogenalkoxy, Carboxyl, C₁-C₄-Alkoxycarbonyl, Benzyloxycarbonyl und/oder Phenyl, wobei die aromatischen Reste ein bis drei der folgenden Substituenten tragen können: Nitro, Cyano, Halogen, C₁-C₄-Alkyl, C₁-C₄-Alkoxy, C₁-C₄-Alkylthio, C₁-C₄-Halogenalkyl, C₁-C₄-Halogenalkyloxy, Carboxyl, C₁-C₄-Alkoxycarbonyl und/oder Benzyloxycarbonyl, C₁-C₄-Alkoxy-C₁-C₆-alkyl;
n 0 bis 3 oder 1 bis 5 für den Fall, daß X Halogen bedeutet,
dadurch gekennzeichnet, daß man Verbindungen der Formel VI 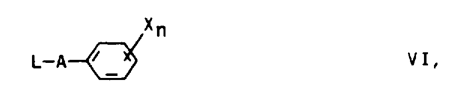 in der L für eine nukleophil verdrängbare Abgangsgruppe steht, umsetzt mit einem Hydroxyimid der Formel VII 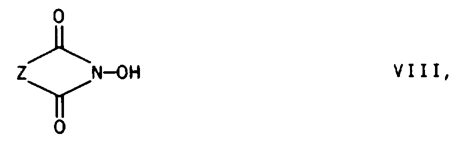 in der Z für einen Phenylen-, Naphthylen, Pyridinylen-, Cyclopentylen-, Cyclohexylen-, Cyclohexenylen-, C₂- bis C₄-Alkenylen- oder C₁- bis C₄-Alkylenrest steht, wobei diese Reste Z unsubstituiert sind oder 1, 2, 3 oder 4 Halogen-, C₁- bis C₄-Alkyl- und/oder C₁- bis C₄-Halogenalkyl-Substituenten tragen können, in Gegenwart eines Lösungsmittels und einer Base bei Temperaturen von 0 bis 140°C umsetzt und aus den erhaltenen Imidethern die Hydroxylamine III mittels Basen oder Säuren freisetzt.

## Claims (Claims for the following Contracting State(s): AT, BE, CH, LI, DE, FR, GB, IT, NL)

1. A cyclohexenone oxime ether of the formula I 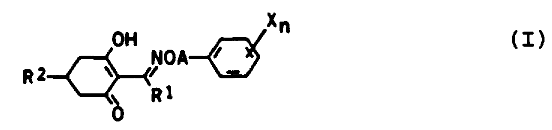 where
R¹ is C₁-C₆-alkyl;
A is a C₃-, C₅- or C₆-alkylene or C₃-, C₅- or C₆-alkenylene chain, where these chains may carry from one to three C₁-C₃-alkyl groups or one methylene group;
X is nitro, cyano, halogen, C₁-C₄-alkyl, C₁-C₄-alkoxy, phenoxy-C₁-C₄-alkylthio, C₁-C₄-haloalkyl, C₁-C₄-haloalkoxy, carboxyl, C₁-C₄-alkoxycarbonyl, benzyloxycarbonyl and/or phenyl, where the aromatic substituents may also carry from one to three radicals selected from the group consisting of nitro, cyano, halogen, C₁-C₄-alkyl, C₁-C₄-alkoxy, C₁-C₄-alkylthio, C₁-C₄-haloalkyl, C₁-C₄-haloalkyloxy, carboxyl, C₁-C₄-alkoxycarbonyl and benzyloxycarbonyl or C₁-C₄-alkoxy-C₁-C₆-alkyl;
n is from 0 to 3, or from 1 to 5 where X is halogen;
R² is C₁-C₄-alkoxy-C₁-C₆-alkyl or C₁-C₄-alkylthio-C₁-C₆-alkyl;
C₃-C₇-cycloalkyl or C₅-C₇-cycloalkenyl, where these groups may carry from one to three radicals selected from the group consisting of C₁-C₄-alkyl, C₁-C₄-alkoxy, C₁-C₄-alkylthio, C₁-C₄-haloalkyl, hydroxyl and halogen;
a 5-membered saturated heterocyclic structure which contains one or two hetero atoms selected from the group consisting of oxygen and sulfur, and which may carry from one to three radicals selected from the group consisting of C₁-C₄-alkyl, C₁-C₄-alkoxy, C₁-C₄-alkylthio and C₁-C₄-haloalkyl,
a saturated or partially or completely unsaturated 6-membered or 7-membered heterocyclic structure containing one or two hetero atoms selected from the group consisting of oxygen and sulfur, where the heterocyclic structure may also carry from one to three radicals selected from the group consisting of hydroxyl, halogen, C₁-C₄-alkyl, C₁-C₄-alkoxy, C₁-C₄-alkylthio and C₁-C₄-haloalkyl, a 5-membered heteroaromatic structure containing one or two nitrogen atoms and one oxygen atom or one sulfur atom, where this ring may also carry from one to three radicals selected from the group consisting of halogen, cyano, C₁-C₄-alkyl, C₁-C₄-alkoxy, C₁-C₄-alkylthio, C₁-C₄-haloalkyl, C₂-C₆-alkenyl, C₂-C₆-alkenyloxy, C₂-C₆-haloalkenyl and C₁-C₄-alkoxy-C₁-C₄-alkyl, or
phenyl or pyridyl, where these groups may also carry from one to three radicals selected from the group consisting of halogen, nitro, cyano, C₁-C₄-alkyl, C₁-C₄-alkoxy, C₁-C₄-alkylthio, C₁-C₄-haloalkyl, C₃-C₆-alkenyloxy, C₃-C₆-alkynyloxy and -NR³R⁴, where
R³ is hydrogen, C₁-C₄-alkyl, C₃-C₆-alkenyl or C₃-C₆-alkynyl and
R⁴ is hydrogen, C₁-C₄-alkyl, C₃-C₆-alkenyl, C₃-C₆-alkynyl, C₁-C₆-acyl or benzoyl, where the aromatic ring may also carry from one to three radicals selected from the group consisting of nitro, cyano, halogen, C₁-C₄-alkyl, C₁-C₄-alkoxy, C₁-C₄-alkylthio and C₁-C₄-haloalkyl,
and its agriculturally useful salts and esters of C₁-C₁₀-carboxylic acids and inorganic acids.

2. A process for the preparation of a compound of the formula I as claimed in claim 1, wherein a corresponding cyclohexenone of the formula II 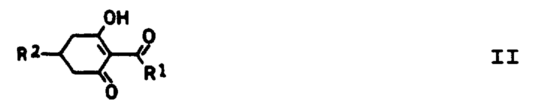 is reacted in a conventional manner, in an inert organic solvent, with a hydroxylamine of the formula III 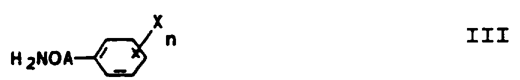 or a salt of the corresponding hydroxylamine.

3. A herbicide containing inert additives and a herbicidal amount of one or more compounds of the formula I as claimed in claim 1.

4. A method for controlling undesirable plant growth, wherein the undesirable plants and/or their habitat are or is treated with a herbicidal amount of a cyclohexenone derivative of the formula I as claimed in claim 1.

5. A hydroxylamine of the formula III 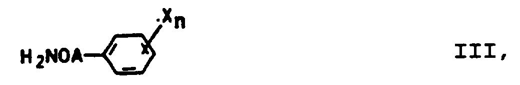 where
A is a C₃-, C₅- or C₆-alkylene or C₃-, C₅- or C₆-alkenylene chain, where these chains may carry from one to three C₁-C₃-alkyl groups or one methylene group;
X is nitro, cyano, halogen, C₁-C₄-alkyl, C₁-C₄-alkoxy, phenoxy, C₁-C₄-alkylthio, C₁-C₄-haloalkyl, C₁-C₄-haloalkoxy, carboxyl, C₁-C₄-alkoxycarbonyl, benzyloxycarbonyl and/or phenyl, where the aromatic radicals may carry from one to three of the following substituents: nitro, cyano, halogen, C₁-C₄-alkyl, C₁-C₄-alkoxy, C₁-C₄-alkylthio, C₁-C₄-haloalkyl, C₁-C₄-haloalkoxy, carboxyl, C₁-C₄-alkoxycarbonyl and/or benzyloxycarbonyl and C₁-C₄-alkoxy-C₁-C₆-alkyl, and
n is from 0 to 3, or from 1 to 5 where X is halogen.

6. A process for the preparation of a hydroxylamine of the formula III as claimed in claim 5, wherein a compound of the formula VI, 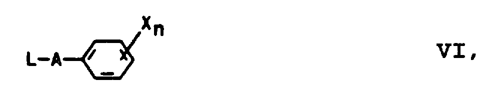 where L is a nucleophilically displaceable leaving group, is reacted with a hydroximide of the formula VIII 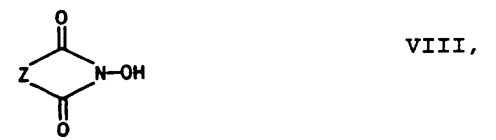 where Z is phenylene, naphthylene, pyridinylene, cyclopentylene, cyclohexylene, cyclohexenylene, C₂-C₄-alkenylene and/or C₁-C₄-alkylene, where these radicals Z are unsubstituted or may carry 1, 2, 3 or 4 halogen, C₁-C₄-alkyl and/or C₁-C₄-haloalkyl substituents, in the presence of a solvent and a base at from 0 to 140°C, and the hydroxylamine III is liberated from the resulting imido ethers by means of a base or acid.

## Claims (Claims for the following Contracting State(s): ES)

1. A process for the preparation of a cyclohexenone oxime ether of the formula I 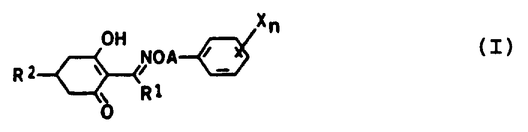 where
R¹ is C₁-C₆-alkyl;
A is a C₃-, C₅- or C₆-alkylene or C₃-, C₅- or C₆-alkenylene chain, where these chains may carry from one to three C₁-C3-alkyl groups or one methylene group;
X is nitro, cyano, halogen, C₁-C₄-alkyl, C₁-C₄-alkoxy, phenoxy-C₁-C₄-alkylthio, C₁-C₄-haloalkyl, C₁-C₄-haloalkoxy, carboxyl, C₁-C₄-alkoxycarbonyl, benzyloxycarbonyl and/or phenyl, where the aromatic substituents may also carry from one to three radicals selected from the group consisting of nitro, cyano, halogen, C₁-C₄-alkyl, C₁-C₄-alkoxy, C₁-C₄-alkylthio, C₁-C₄-haloalkyl, C₁-C₄-haloalkyloxy, carboxyl, C₁-C₄-alkoxycarbonyl, benzyloxycarbonyl and C₁-C₄-alkoxy-C₁-C₆-alkyl;
n is from 0 to 3, or from 1 to 5 where X is halogen;
R² is C₁-C₄-alkoxy-C₁-C₆-alkyl or C₁-C₄-alkylthio-C₁-C₆-alkyl;
C₃-C₇-cycloalkyl or C₅-C₇-cycloalkenyl, where these groups may carry from one to three radicals selected from the group consisting of C₁-C₄-alkyl, C₁-C₄-alkoxy, C₁-C₄-alkylthio, C₁-C₄-haloalkyl, hydroxyl and halogen;
a 5-membered saturated heterocyclic structure which contains one or two hetero atoms selected from the group consisting of oxygen and sulfur, and which may carry from one to three radicals selected from the group consisting of Cₗ-C₄-alkyl, C₁-C₄-alkoxy, C₁-C₄-alkylthio and C₁-C₄-haloalkyl,
a saturated or partially or completely unsaturated 6-membered or 7-membered heterocyclic structure containing one or two hetero atoms selected from the group consisting of oxygen and sulfur, where the heterocyclic structure may also carry from one to three radicals selected from the group consisting of hydroxyl, halogen, C₁-C₄-alkyl, C₁-C₄-alkoxy, C₁-C₄-alkylthio and C₁-C₄-haloalkyl, a 5-membered heteroaromatic structure containing one or two nitrogen atoms and one oxygen atom or one sulfur atom, where this ring may also carry from one to three radicals selected from the group consisting of halogen, cyano, C₁-C₄-alkyl, C₁-C₄-alkoxy, C₁-C₄-alkylthio, C₁-C₄-haloalkyl, C₂-C₆-alkenyl, C₂-C₆-alkenyloxy, C₂-C₆-haloalkenyl and C₁-C₄-alkoxy-C₁-C₄-alkyl, or phenyl or pyridyl, where these groups may also carry from one to three radicals selected from the group consisting of halogen, nitro, cyano, C₁-C₄-alkyl, C₁-C₄-alkoxy, C₁-C₄-alkylthio, C₁-C₄-haloalkyl, C₃-C₆-alkenyloxy, C₃-C₆-alkynyloxy and -NR³R⁴, where
R³ is hydrogen, C₁-C₄-alkyl, C₃-C₆-alkenyl or C₃-C₆-alkynyl and
R⁴ is hydrogen, C₁-C₄-alkyl, C₃-C₆-alkenyl, C₃-C₆-alkynyl, C₁-C₆-acyl or benzoyl, where the aromatic ring may also carry from one to three radicals selected from the group consisting of nitro, cyano, halogen, C₁-C₄-alkyl, C₁-C₄-alkoxy, C₁-C₄-alkylthio and C₁-C₄-haloalkyl,
and its agriculturally useful salts and esters of C₁-C₁₀-carboxylic acids and inorganic acids, wherein a corresponding cyclohexenone of the formula II 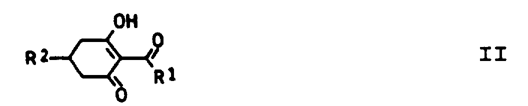
is reacted in a conventional manner, in an inert organic solvent, with a hydroxylamine of the formula III 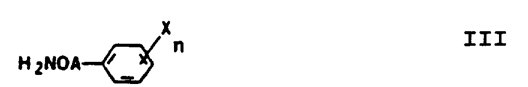 or a salt of the corresponding hydroxylamine.

2. A herbicide containing inert additives and a herbicidal amount of one or more compounds of the formula I as claimed in claim 1.

3. A method for controlling undesirable plant growth, wherein the undesirable plants and/or their habitat are or is treated with a herbicidal amount of a cyclohexenone derivative of the formula I as claimed in claim 1.

4. A process for the preparation of a hydroxylamine of the formula III 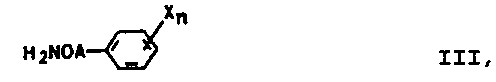 where
A is a C₃-, C₅- or C₆-alkylene or C₃-, C₅- or C₆-alkenylene chain, where these chains may carry from one to three C₁-C₃-alkyl groups or one methylene group;
X is nitro, cyano, halogen, C₁-C₄-alkyl, C₁-C₄-alkoxy, phenoxy, C₁-C₄-alkylthio, C₁-C₄-haloalkyl, C₁-C₄-haloalkoxy, carboxyl, C₁-C₄-alkoxycarbonyl, benzyloxycarbonyl and/or phenyl, where the aromatic radicals may carry from one to three of the following substituents: nitro, cyano, halogen, C₁-C₄-alkyl, C₁-C₄-alkoxy, C₁-C₄-alkylthio, C₁-C₄-haloalkyl, C₁-C₄-haloalkoxy, carboxyl, C₁-C₄-alkoxycarbonyl and/or benzyloxycarbonyl and C₁-C₄-alkoxy-C₁-C₆-alkyl, and
n is from 0 to 3, or from 1 to 5 where X is halogen, wherein a compound of the formula VI, 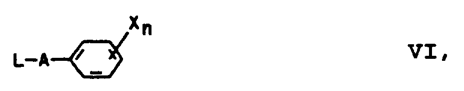 where L is a nucleophilically displaceable leaving group, is reacted with a hydroximide of the formula VIII 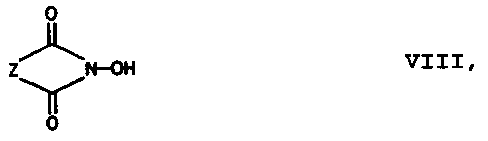 where Z is phenylene, naphthylene, pyridinylene, cyclopentylene, cyclohexylene, cyclohexenylene, C₂-C₄-alkenylene or C₁-C₄-alkylene, where these radicals Z are unsubstituted or may carry 1, 2, 3 or 4 halogen, C₁-C₄-alkyl and/or C₁-C₄-haloalkyl substituents, in the presence of a solvent and a base at from 0 to 140°C, and the hydroxylamine III is liberated from the resulting imido ethers by means of a base or acid.

## Revendications (Revendications pour l'(les) Etat(s) contractant(s) suivant(s): AT, BE, CH, LI, DE, FR, GB, IT, NL)

1. Ethers de cyclohexénonoximes de formule générale I 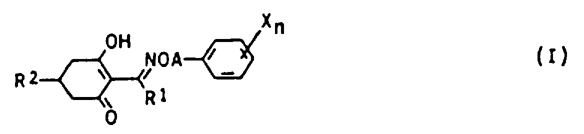 dans laquelle les symboles ont les significations suivantes :
R¹ représente un groupe alkyle en C1-C6;
A représente une chaîne alkylène en C3, en C5, en C6, ou alcénylène en C3, C5, C6, ces chaînes pouvant porter un à trois groupes alkyle en C1-C3 ou un groupe méthylène ;
X représente un groupe nitro, cyano, un halogène, un groupe alkyle en C1-C4, alcoxy en C1-C4, phénoxy-alkylthio en C1-C4, halogénoalkyle en C1-C4, halogénoalcoxy en C1-C4, carboxyle, (alcoxy en C1-C4)carbonyle, benzyloxycarbonyle et/ou phényle, les groupes aromatiques pouvant encore porter un à trois substituants choisis parmi les groupes nitro, cyano, les halogènes, les groupes alkyle en C1-C4, alcoxy en C1-C4, alkylthio en C1-C4, halogénoalkyle en C1-C4, halogénoalcoxy en C1-C4, carboxyle, (alcoxy en Cl-C4)carbonyle et benzyloxycarbonyle, (alcoxy en C1-C4)alkyle en C1-C6;
n est un nombre allant de 0 à 3 ou de 1 à 5 lorsque X représente un halogène ;
R² représente un groupe (alcoxy en C1-C4)alkyle en C1-C6 ou (alkylthio en C1-C4)alkyle en C1-C6 ;
un groupe cycloalkyle en C3-C7 ou un groupe cycloalcényle en C5-C7, ces groupes pouvant porter un à trois substituants choisis parmi les groupes alkyle en C1-C4, alcoxy en C1-C4, alkylthio en C1-C4, halogénoalkyle en C1-C4, hydroxy et les halogènes ;
un hétérocycle saturé à cinq chaînons contenant un ou deux hétéroatomes choisis parmi l'oxygène et le soufre et qui peut porter un à trois substituants choisis parmi les groupes alkyle en C1-C4, alcoxy en C1-C4, alkylthio en C1-C4 et halogénoalkyle en C1-C4 ;
un hétérocycle saturé ou partiellement ou totalement insaturé, à six ou sept chaînons, contenant un ou deux hétéroatomes choisis parmi l'oxygène et le soufre, l'hétérocycle pouvant encore porter un à trois substituants choisis parmi les groupes hydroxy, les halogènes, les groupes alkyle en C1-C4, alcoxy en C1-C4, alkylthio en C1-C4 et halogénoalkyle en C1-C4,
un groupe hétéroaromatique à cinq chaînons contenant un à deux atomes d'azote et un atome d'oxygène ou un atome de soufre, ce cycle pouvant encore porter un à trois substituants choisis parmi les halogènes, les groupes cyano, alkyle en C1-C4, alcoxy en C1-C4, alkylthio en C1-C4, halogénoalkyle en C1-C4, alcényle en C2-C6, alcényloxy en C2-C6, halogénoalcényle en C2-C6 et (alcoxy en C1-C4)alkyle en C1-C4,
un groupe phényle ou un groupe pyridyle, ces groupes pouvant encore porter un à trois substituants choisis parmi les halogènes, les groupes nitro, cyano, alkyle en C1-C4, alcoxy en C1-C4, alkylthio en C1-C4, halogénoalkyle en C1-C4, alcényloxy en C3-C6, alcynyloxy en C3-C6 et -NR³R⁴ dans lequel
R³ représente l'hydrogène, un groupe alkyle en C1-C4, alcényle en C3-C6 ou alcynyle en C3-C6 et
R⁴ représente l'hydrogène, un groupe alkyle en C1-C4, alcényle en C3-C6, alcynyle en C3-C6, acyle en C1-C6 ou benzoyle, le noyau aromatique pouvant encore porter un à trois substituants choisis parmi les groupes nitro, cyano, les halogènes, les groupes alkyle en C1-C4, alcoxy en C1-C4, alkylthio en C1-C4 et halogénoalkyle en C1-C4,
et leurs sels et esters d'acides carboxyliques en C1-C10 et d'acides minéraux aptes à des applications agricoles.

2. Procédé de préparation des composés de formule I de la revendication 1, caractérisé par le fait que l'on fait réagir une cyclohexénone correspondante de formule II 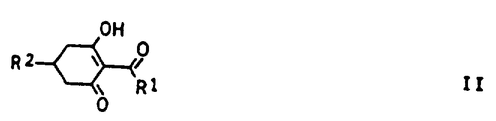 de manière connue en soi, dans un solvant organique inerte, avec une hydroxylamine de formule III 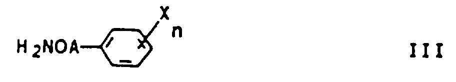 ou avec un sel de l'hydroxylamine correspondante.

3. Produit herbicide contenant des additifs inertes et une quantité herbicide efficace d'au moins un composé de formule I de la revendication 1.

4. Procédé pour combattre les croissances de végétaux indésirables, caractérisé par le fait que l'on traite les végétaux indésirables et/ou leur habitat par une quantité herbicide efficace d'un dérivé de cyclohexénone de formule I de la revendication 1.

5. Hydroxylamines de formule III 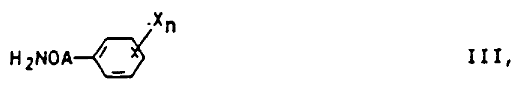 dans laquelle A et Xₙ ont les significations suivantes :
A représente une chaîne alkylène en C3, en C5 ou en C6 ou alcénylène en C3, en C5 ou en C6, ces chaînes pouvant encore porter un à trois groupes alkyle en C1-C3 ou un groupe méthylène ;
X représente un groupe nitro, cyano, un halogène, un groupe alkyle en C1-C4, alcoxy en C1-C4, phénoxy, alkylthio en C1-C4, halogénoalkyle en C1-C4, halogénoalcoxy en C1-C4, carboxyle, (alcoxy en C1-C4)carbonyle, benzyloxycarbonyle et/ou phényle, les radicaux aromatiques pouvant porter un à trois des substituants suivants : nitro, cyano, halogéno, alkyle en C1-C4, alcoxy en C1-C4, alkylthio en C1-C4, halogénoalkyle en C1-C4, halogénoalcoxy en C1-C4, carboxyle, (alcoxy en C1-C4)carbonyle et/ou benzyloxycarbonyle, (alcoxy en C1-C4)alkyle en C1-C6 ;
n est un nombre allant de 0 à 3 ou de 1 à 5 lorsque X représente un halogène.

6. Procédé de préparation des hydroxylamines de formule générale III de la revendication 5, caractérisé par le fait que l'on fait réagir des composés de formule VI 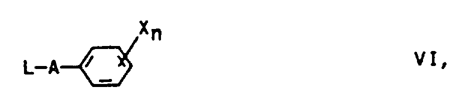 dans laquelle L représente un groupe éliminable nucléophile, avec un hydroxyimide de formule VII 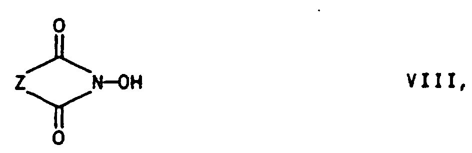 dans laquelle Z représente un groupe phénylène, naphtylène, pyridinylène, cyclopentylène, cyclohexylène, cyclohexénylène, alcénylène en C2-C4 ou alkylène en C1-C4, ces groupes Z pouvant être non substitués ou porter un, deux, trois ou quatre substituants halogéno, alkyle en C1-C4 et/ou halogénoalkyle en C1-C4, en présence d'un solvant et d'une base, à des températures de 0 à 140°C, ce qui donne des éthers-imides d'où l'on libère les hydroxyamines III à l'aide de bases ou d'acides.

## Revendications (Revendications pour l'(les) Etat(s) contractant(s) suivant(s): ES)

1. Procédé de préparation des éthers de cyclohexénonoximes de formule générale I 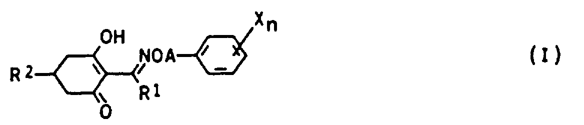 dans laquelle les symboles ont les significations suivantes :
R¹ représente un groupe alkyle en C1-C6 ;
A représente une chaîne alkylène en C3, en C5, en C6, ou alcénylène en C3, C5, C6, ces chaînes pouvant porter un à trois groupes alkyle en C1-C3 ou un groupe méthylène ;
X représente un groupe nitro, cyano, un halogène, un groupe alkyle en C1-C4, alcoxy en C1-C4, phénoxy-alkylthio en C1-C4, halogénoalkyle en C1-C4, halogénoalcoxy en C1-C4, carboxyle, (alcoxy en C1-C4)carbonyle, benzyloxycarbonyle et/ou phényle, les groupes aromatiques pouvant encore porter un à trois substituants choisis parmi les groupes nitro, cyano, les halogènes, les groupes alkyle en C1-C4, alcoxy en C1-C4, alkylthio en C1-C4, halogénoalkyle en C1-C4, halogénoalcoxy en C1-C4, carboxyle, (alcoxy en Cl-C4)carbonyle et benzyloxycarbonyle, (alcoxy en C1-C4)alkyle en C1-C6 ;
n est un nombre allant de 0 à 3 ou de 1 à 5 lorsque X représente un halogène ;
R² représente un groupe (alcoxy en C1-C4)alkyle en C1-C6 ou (alkylthio en C1-C4)alkyle en C1-C6 ;
un groupe cycloalkyle en C3-C7 ou un groupe cycloalcényle en C5-C7, ces groupes pouvant porter un à trois substituants choisis parmi les groupes alkyle en C1-C4, alcoxy en C1-C4, alkylthio en C1-C4, halogénoalkyle en C1-C4, hydroxy et les halogènes ;
un hétérocycle saturé à cinq chaînons contenant un ou deux hétéroatomes choisis parmi l'oxygène et le soufre et qui peut porter un à trois substituants choisis parmi les groupes alkyle en C1-C4, alcoxy en C1-C4, alkylthio en C1-C4 et halogénoalkyle en C1-C4 ;
un hétérocycle saturé ou partiellement ou totalement insaturé, à six ou sept chaînons, contenant un ou deux hétéroatomes choisis parmi l'oxygène et le soufre, l'hétérocycle pouvant encore porter un à trois substituants choisis parmi les groupes hydroxy, les halogènes, les groupes alkyle en C1-C4, alcoxy en C1-C4, alkylthio en C1-C4 et halogénoalkyle en C1-C4,
un groupe hétéroaromatique à cinq chaînons contenant un à deux atomes d'azote et un atome d'oxygène ou un atome de soufre, ce cycle pouvant encore porter un à trois substituants choisis parmi les halogènes, les groupes cyano, alkyle en C1-C4, alcoxy en C1-C4, alkylthio en C1-C4, halogénoalkyle en C1-C4, alcényle en C2-C6, alcényloxy en C2-C6, halogénoalcényle en C2-C6 et (alcoxy en C1-C4)alkyle en C1-C4,
un groupe phényle ou un groupe pyridyle, ces groupes pouvant encore porter un à trois substituants choisis parmi les halogènes, les groupes nitro, cyano, alkyle en C1-C4, alcoxy en C1-C4, alkylthio en C1-C4, halogénoalkyle en C1-C4, alcényloxy en C3-C6, alcynyloxy en C3-C6 et -NR³R⁴ dans lequel
R³ représente l'hydrogène, un groupe alkyle en C1-C4, alcényle en C3-C6 ou alcynyle en C3-C6 et
R⁴ représente l'hydrogène, un groupe alkyle en C1-C4, alcényle en C3-C6, alcynyle en C3-C6, acyle en C1-C6 ou benzoyle, le noyau aromatique pouvant encore porter un à trois substituants choisis parmi les groupes nitro, cyano, les halogènes, les groupes alkyle en C1-C4, alcoxy en C1-C4, alkylthio en C1-C4 et halogénoalkyle en C1-C4,
et leurs sels et esters d'acides carboxyliques en C1-C10 et d'acides minéraux aptes à des applications agricoles, caractérisé par le fait qu'on fait réagir, de manière connue, une cyclohexénone de formule II 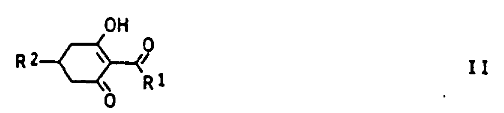 dans un diluant organique inerte avec une hydroxylamine de formule III 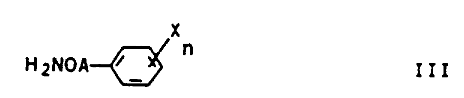 ou un sel de l'hydroxylamine correspondante.

2. Produit herbicide contenant des additifs inertes et une quantité herbicide efficace d'au moins un composé de formule I de la revendication 1.

3. Procédé pour combattre les croissances de végétaux indésirables, caractérisé par le fait que l'on traite les végétaux indésirables et/ou leur habitat par une quantité herbicide efficace d'un dérivé de cyclohexénone de formule I de la revendication 1.

4. Procédé de préparation des hydroxylamines de formule générale III 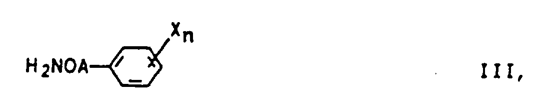 dans laquelle
A représente une chaîne alkylène en C3, en C5 ou en C6 ou alcénylène en C3, en C5 ou en C6, ces chaînes pouvant encore porter un à trois groupes alkyle en C1-C3 ou un groupe méthylène ;
X représente un groupe nitro, cyano, un halogène, un groupe alkyle en C1-C4, alcoxy en C1-C4, phénoxy, alkylthio en C1-C4, halogénoalkyle en C1-C4, halogénoalcoxy en C1-C4, carboxyle, (alcoxy en C1-C4)carbonyle, benzyloxycarbonyle et/ou phényle, les radicaux aromatiques pouvant porter un à trois des substituants suivants : nitro, cyano, halogéno, alkyle en C1-C4, alcoxy en C1-C4, alkylthio en C1-C4, halogénoalkyle en C1-C4, halogénoalcoxy en C1-C4, carboxyle, (alcoxy en C1-C4)carbonyle et/ou benzyloxycarbonyle, (alcoxy en C1-C4)alkyle en C1-C6 ;
n est un nombre allant de 0 à 3 ou de 1 à 5 lorsque X représente un halogène,
caractérisé par le fait que l'on fait réagir des composés de formule VI 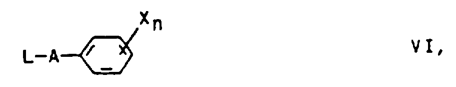 dans laquelle L représente un groupe éliminable nucléophile, avec un hydroxyimide de formule VII 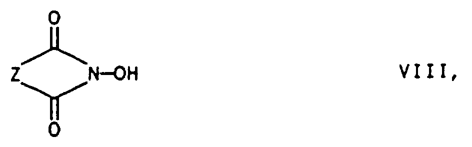 dans laquelle Z représente un groupe phénylène, naphtylène, pyridinylène, cyclopentylène, cyclohexylène, cyclohexénylène, alcénylène en C2-C4 ou alkylène en C1-C4, ces groupes Z pouvant être non substitués ou porter un, deux, trois ou quatre substituants halogéno, alkyle en C1-C4 et/ou halogénoalkyle en C1-C4, en présence d'un solvant et d'une base, à des températures de 0 à 140°C, ce qui donne des éthers-imides d'où l'on libère les hydroxyamines III à l'aide de bases ou d'acides.
